(19) **Europäisches Patentamt / European Patent Office / Office européen des brevets**

(11) **EP 4 624 936 A1**

(12) # EUROPEAN PATENT APPLICATION
published in accordance with Art. 153(4) EPC

(43) Date of publication:
**01.10.2025 Bulletin 2025/40**

(21) Application number: **23894409.4**

(22) Date of filing: **07.11.2023**

(51) International Patent Classification (IPC):
*G01N 35/08* (2006.01)    *B81B 1/00* (2006.01)
*B81C 1/00* (2006.01)    *G01N 37/00* (2006.01)

(52) Cooperative Patent Classification (CPC):
**B81B 1/00; B81C 1/00; G01N 35/08; G01N 37/00**

(86) International application number:
**PCT/JP2023/039970**

(87) International publication number:
**WO 2024/111397 (30.05.2024 Gazette 2024/22)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **25.11.2022 JP 2022188117**
**02.11.2023 JP 2023188110**

(71) Applicant: **Dexerials Corporation**
**Shimotsuke-shi, Tochigi 323-0194 (JP)**

(72) Inventor: **MONJU, Takuya**
**Shimotsuke-shi, Tochigi 323-0194 (JP)**

(74) Representative: **Ter Meer Steinmeister & Partner Patentanwälte mbB**
**Nymphenburger Straße 4**
**80335 München (DE)**

(54) **LAMINATE STRUCTURE AND MANUFACTURING METHOD THEREOF, INSPECTION DEVICE, AND SHEET-LIKE STRUCTURE-USE ADHESIVE SHEET AND METHOD OF USE THEREOF**

(57) A laminate structure includes: a first sheet-like structure having a porous structure serving as a flow path exposed at a surface of the first sheet-like structure; a second sheet-like structure having a porous structure serving as a flow path exposed at a surface of the second sheet-like structure; and an adhesive layer that is arranged between the first sheet-like structure and the second sheet-like structure and is in contact with part of a surface of the porous structure of the first sheet-like structure and part of a surface of the porous structure of the second sheet-like structure such that the porous structure of the first sheet-like structure and the porous structure of the second sheet-like structure face each other, and the first sheet-like structure and the second sheet-like structure are not separated.

FIG.2B

**Description**

TECHNICAL FIELD

The present invention relates to a laminate structure, a method of producing the laminate structure, an inspection device, a thermal adhesive sheet for a sheet-like structure, and a method of using the thermal adhesive sheet for the sheet-like structure.

BACKGROUND ART

Inspection devices that enable simple and rapid diagnosis in daily life or clinical practice have been developed. A representative example of the inspection device is a pregnancy test kit. When a test liquid including a target substance, such as an antigen, is introduced into the inspection device, the test liquid flows through a flow path within the inspection device. Then, a labeling medium such as an antibody, which has been disposed in the flow path in advance, reacts with the target substance included in the testing liquid to develop a color (to generate a color) so that the presence of the target substance can be confirmed.

Inspection chips, which are one example of the inspection devices, are also referred to as "u-PADs (microfluidic paper-based analytical devices)." The inspection chips have many advantages, such as (1) low cost, (2) pumpless, (3) no need of complex devices, and (4) easy disposal, and therefore studies for improving the inspection chips have been conducted worldwide.

Various inspection chips have been already reported. Examples of the reported inspection chips include a microfluidic device in which a flow path or a reaction spot is formed on paper serving as a substrate using a wax printer or an inkjet printer (see NON-PATENT DOCUMENT 1).

Moreover, inspection chips in which a three-dimensional flow path is formed for the purpose of conducting a multistage reaction, or imparting an effect of controlling a flow of a specimen in the flow path are also proposed. For example, an inspection chip in which a three-dimensional flow path is formed in one sheet of filter paper (see Patent Document 1), an inspection chip obtained by bonding multiple sheets of chips in which flow paths are formed together with a double-sided tape (see Non-Patent Document 2), an inspection chip obtained by bonding multiple sheets of chips in which flow paths are formed together by a stapler (see Patent Document 2), and the like are disclosed.

CITATION LIST

PATENT DOCUMENTS

Patent Document 1: Japanese Unexamined Patent Application Publication No. 2021-175970
Patent Document 2: Japanese Unexamined Patent Application Publication No. 2022-018036

NON-PATENT DOCUMENTS

NON-PATENT DOCUMENT 1: Whiteside et al., Analytical Chemistry, Vol. 82, No. 1, January 1, 2010
NON-PATENT DOCUMENT 2: Quoc Trung Hua et al., analytical sciences, Vol. 35, April, 2019

SUMMARY OF THE INVENTION

TECHNICAL PROBLEM

However, a flow path in the inspection device including the above-described technology described in Non-Patent Document 1 can be only extended in in a planar direction, and therefore it is difficult to achieve a complex structure of the flow path. Thus, the above inspection device is not suitable as an inspection device for a multistage reaction. Similarly, the device of the invention disclosed in Patent Document 1 above has a limitation in a three-dimensional structure that can be formed within one sheet of filter paper, and therefore there is a need for further improvement in complexity as an inspection device for a multistage reaction. The inspection device including the above-described technology described in Non-Patent Document 1 is formed by bonding multiple substrates in each of which a flow path is formed together with a double-sided tape. Therefore, it is necessary to fill a gap formed between the substrates, corresponding to the thickness of the double-sided tape, and the assembly process of the inspection device is complicated. The device including the above-described invention described in Patent Document 2 is formed by bonding multiple substrates in each of which a flow path is formed together by a stapler. Therefore, it is difficult to reliably bond the substrates, and there is a concern in stable flowability of a liquid.

The present invention aims to solve the above-described various problems existing in the related art and to achieve the following object. That is, an object of the present invention is to provide a laminate structure that can make bonding easily, while ensuring sufficient flowability.

SOLUTION TO THE PROBLEM

Means for solving the above problems are as follows.

<1> A laminate structure including:

a first sheet-like structure having a porous structure serving as a flow path, the porous structure being exposed at a surface of the first sheet-like structure;
a second sheet-like structure having a porous structure serving as a flow path, the porous structure being exposed at a surface of the second sheet-like structure; and
an adhesive layer that is arranged between the first sheet-like structure and the second sheet-like structure and is in contact with part of a surface of the porous structure of the first sheet-like structure and part of a surface of the porous structure of the second sheet-like structure in a manner such that the porous structure of the first sheet-like structure and the porous structure of the second sheet-like structure face each other, and the first sheet-like structure and the second sheet-like structure are not separated.

<2> The laminate structure according to <1>,
wherein the adhesive layer is water resistant.
<3> The laminate structure according to <1> or <2>,
wherein the adhesive layer is in a shape of parallel lines.
<4> The laminate structure according to any one of <1> to <3>,
wherein the adhesive layer is in a mesh form.
<5> The laminate structure according to any one of <1> to <4>,
wherein a ratio of a connection area of the flow path to an opening area per opening of the adhesive layer is 5 or greater.
<6> The laminate structure according to any one of <1> to <5>,
wherein the adhesive layer includes a thermal adhesive.
<7> The laminate structure according to <6>,
wherein the laminate structure includes a thermal adhesive sheet that includes a substrate having multiple openings, and the adhesive layer in which the thermal adhesive is arranged in a mesh form on the substrate.
<8> The laminate structure according to <7>,
wherein the substrate is in a mesh form.
<9> An inspection device including:
the laminate structure of any one of <1> to <8>.
<10> A method of producing the laminate structure of any one of <1> to <8>, the method including:

an adhesive layer formation step of arranging a thermal adhesive in a shape of parallel lines to form the adhesive layer on a release substrate;
a transfer step of transferring the adhesive layer onto the first sheet-like structure having the porous structure serving as a flow path exposed at the surface of the first sheet-like structure; and
a bonding step of removing the release substrate, and bonding the second sheet-like structure having the porous structure serving as a flow path exposed at the surface of the second sheet-like structure.

<11> The method of producing the laminate structure according to <10>,
wherein the adhesive layer formation step includes arranging the thermal adhesive in a mesh form to form the adhesive layer.
<12> A method of producing the laminate structure of any one of <1> to <8>, the method including:

a transfer step of transferring a thermal adhesive sheet onto the first sheet-like structure having the porous structure serving as a flow path exposed at the surface of the first sheet-like structure, the thermal adhesive sheet including a substrate having multiple openings, and the adhesive layer in which a thermal adhesive is arranged in a shape of parallel lines on the substrate; and

a bonding step of bonding the second sheet-like structure having the porous structure serving as a flow path exposed at the surface of the second sheet-like structure.

<13> The method of producing the laminate structure according to <12>,
wherein the thermal adhesive sheet includes the adhesive layer in which the thermal adhesive is arranged in a mesh form.
<14> A thermal adhesive sheet for a sheet-like structure including:

a substrate having multiple openings; and
an adhesive layer in which a thermal adhesive is arranged in a mesh form on the substrate,

wherein the thermal adhesive sheet for the sheet-like structure is used for a sheet-like structure having a porous structure serving as a flow path exposed at a surface of the sheet-like structure.
<15> A method of using a thermal adhesive sheet for a sheet-like structure, the method including:
using the thermal adhesive sheet for the sheet-like structure for a sheet-like structure having a porous structure serving as a flow path exposed at a surface of the sheet-like structure,
wherein the thermal adhesive sheet for the sheet-like structure includes a substrate having multiple openings, and an adhesive layer in which a thermal adhesive is arranged in a mesh form on the substrate.

EFFECTS OF THE INVENTION

According to the present invention, a laminate structure that can make bonding easy while ensuring sufficient flowability can be provided.

BRIEF DESCRIPTION OF THE DRAWINGS

[Fig. 1] Fig. 1 is a schematic cross-sectional view illustrating an example of a laminate structure.
[Fig. 2A] Fig. 2A is a schematic cross-sectional view of a laminate structure of a first embodiment.
[Fig. 2B] Fig. 2B is a schematic perspective view of layers constituting the laminate structure of the first embodiment.
[Fig. 3A] Fig. 3A is a schematic perspective view of a first sheet-like structure of the laminate structure of the first embodiment of Fig. 2A as viewed in an $\alpha$ direction.
[Fig. 3B] Fig. 3B is a schematic perspective view of the first sheet-like structure of the laminate structure of the first embodiment of Fig. 2A as viewed in a $\beta$ direction.
[Fig. 3C] Fig. 3C is a schematic plan view of the first sheet-like structure of the laminate structure of the first embodiment of Fig. 2A as viewed in the $\alpha$ direction.
[Fig. 3D] Fig. 3D is a schematic plan view of the first sheet-like structure of the laminate structure of the first embodiment of Fig. 2A as viewed in the $\beta$ direction.
[Fig. 4] Fig. 4 is a schematic cross-sectional view of the first sheet-like structure of Fig. 3C cut along the line a-a'.
[Fig. 5A] Fig. 5A is a schematic plan view illustrating an example of an adhesive layer of the laminate structure of the first embodiment as viewed in the $\alpha$ direction.
[Fig. 5B] Fig. 5B is a schematic plan view illustrating another example of the adhesive layer of the laminate structure of the first embodiment as viewed in the $\alpha$ direction.
[Fig. 6A] Fig. 6A is a schematic perspective view for explaining an example of a distribution of the adhesive layer in the laminate structure of the first embodiment.
[Fig. 6B] Fig. 6B is a schematic perspective view for explaining another example of the distribution of the adhesive layer in the laminate structure of the first embodiment.
[Fig. 7] Fig. 7 is an image illustrating an example of a thermal adhesive sheet.
[Fig. 8] Fig. 8 is a schematic cross-sectional view of a laminate structure of a second embodiment.
[Fig. 9A] Fig. 9A is a schematic plan view of a first sheet-like structure of the laminate structure of the second embodiment of Fig. 8 as viewed in an $\alpha$ direction.
[Fig. 9B] Fig. 9B is a schematic plan view of the first sheet-like structure of the laminate structure of the second embodiment of Fig. 8 as viewed in a $\beta$ direction.
[Fig. 10] Fig. 10 is a schematic cross-sectional view of the first sheet-like structure of Fig. 9A cut along the line **b-b'.**
[Fig. 11] Fig. 11 is a schematic cross-sectional view of a laminate structure of a third embodiment.
[Fig. 12A] Fig. 12A is a schematic plan view of a first sheet-like structure of the laminate structure of the third embodiment of Fig. 11 as viewed in an $\alpha$ direction.

[Fig. 12B] Fig. 12B is a schematic plan view of the first sheet-like structure of the laminate structure of the third embodiment of Fig. 11 as viewed in a $\beta$ direction.

[Fig. 13] Fig. 13 is a schematic cross-sectional view of the first sheet-like structure of Fig. 12A cut along the line c-c'.

[Fig. 14A] Fig. 14A is a schematic plan view of a first sheet-like structure of a fourth embodiment in a laminate of the fourth embodiment as viewed in an $\alpha$ direction.

[Fig. 14B] Fig. 14B is a schematic plan view of the first sheet-like structure of the fourth embodiment in the laminate structure of the fourth embodiment as viewed in a $\beta$ direction.

[Fig. 15A] Fig. 15A is a schematic plan view of a first sheet-like structure of a fifth embodiment in a laminate structure of the fifth embodiment as viewed in an $\alpha$ direction.

[Fig. 15B] Fig. 15B is a schematic plan view of the first sheet-like structure of the fifth embodiment in the laminate structure of the fifth embodiment as viewed in a $\beta$ direction.

[Fig. 16A] Fig. 16A is a schematic plan view of a first sheet-like structure of a sixth embodiment in a laminate structure of the sixth embodiment as viewed in an $\alpha$ direction.

[Fig. 16B] Fig. 16B is a schematic plan view of the first sheet-like structure of the sixth embodiment in the laminate structure of the sixth embodiment as viewed in a $\beta$ direction.

[Fig. 17A] Fig. 17A is a schematic plan view of a first sheet-like structure of a seventh embodiment in a laminate structure of the seventh embodiment as viewed in an $\alpha$ direction.

[Fig. 17B] Fig. 17B is a schematic plan view of the first sheet-like structure of the seventh embodiment in the laminate structure of the seventh embodiment as viewed in a $\beta$ direction.

[Fig. 18A] Fig. 18A is a schematic plan view of a first sheet-like structure of an eighth embodiment in a laminate structure of the eighth embodiment as viewed in an $\alpha$ direction.

[Fig. 18B] Fig. 18B is a schematic plan view of the first sheet-like structure of the eighth embodiment in the laminate structure of the eighth embodiment as viewed in a $\beta$ direction.

[Fig. 19A] Fig. 19A is a schematic plan view of a first sheet-like structure of a ninth embodiment in a laminate structure of the ninth embodiment as viewed in an $\alpha$ direction.

[Fig. 19B] Fig. 19B is a schematic plan view of the first sheet-like structure of the ninth embodiment in the laminate structure of the ninth embodiment as viewed in a $\beta$ direction.

[Fig. 20A] Fig. 20A is an explanatory view for explaining a specific flow path shape in Examples.

[Fig. 20B] Fig. 20B is an explanatory view for explaining a specific flow path shape in Examples.

[Fig. 20C] Fig. 20C is an explanatory view for explaining a missing flow path shape in Examples.

[Fig. 20D] Fig. 20D is an explanatory view for explaining a missing flow path shape in Examples.

[Fig. 21A] Fig. 21A is an explanatory view for explaining a single-side or double-side printed flow path in Examples.

[Fig. 21B] Fig. 21B is a cross-sectional photograph of the single-side printed flow path of Fig. 21A cut along the line d-d'.

[Fig. 21C] Fig. 21C is a cross-sectional photograph of the double-side printed flow path of Fig. 21A cut along the line d-d'.

[Fig. 22] Fig. 22 is an explanatory view for explaining a doughnut-like flow path shape in Examples.

DESCRIPTION OF EMBODIMENTS

(Laminate structure)

The laminate structure of the present invention includes a first sheet-like structure, a second sheet-like structure, and an adhesive layer. The first sheet-like structure has a porous structure serving as a flow path, and the porous structure is exposed at a surface of the first sheet-like structure. The second sheet-like structure has a porous structure serving as a flow path, and the porous structure is exposed at a surface of the second sheet-like structure. The adhesive layer is arranged between the first sheet-like structure and the second sheet-like structure to be in contact with part of a surface of the porous surface of the first sheet-like structure and part of a surface of the porous surface of the second sheet-like structure such that the porous structure of the first sheet-like structure and the porous structure of the second sheet-like structure face each other, and the first sheet-like structure and the second sheet-like structure are not separated.

The present invention will be described in detail based on several embodiments hereinafter, but the present invention is not limited to the following descriptions of the embodiments in any way.

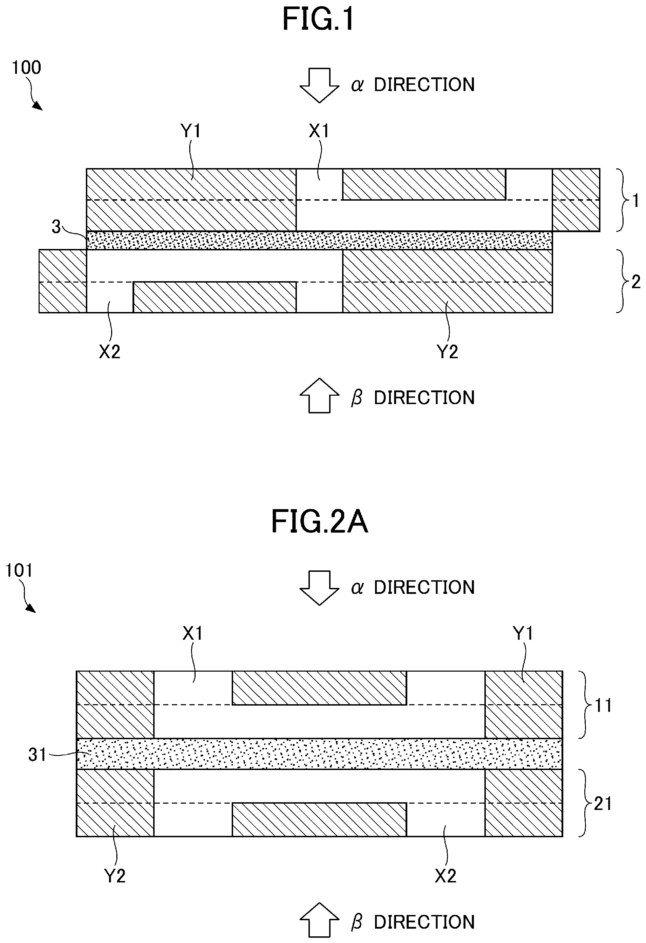

Fig. 1 is a schematic cross-sectional view illustrating an example of the laminate structure.

The laminate structure 100 includes a first sheet-like structure 1 including a flow path X1 and a non-flow path Y1, a second sheet-like structure 2 including a flow path X2 and a non-flow path Y2, and an adhesive layer 3. Although the details will be described later, the adhesive layer 3 is arranged between the first sheet-like structure and the second sheet-like structure to be in contact with part of surfaces of porous structures to be flow paths in a manner such that the flow path (porous structure) of the first sheet-like structure and the flow path (porous structure) of the second sheet-like structure face each other, and the first sheet-like structure and the second sheet-like structure are not separated.

Since the laminate structure 100 has the above configuration, sufficient flowability can be ensured without a complex assemble process as used in the related art.

For the first sheet-like structure and the second sheet-like structure, sheet-like structures having the same flow paths may be used, or sheet-like structures having mutually different flow paths may be used.

Since the second sheet-like structure can have the same configuration as the first sheet-like structure, only the configuration of the first sheet-like structure may be described hereinafter, and descriptions of the second sheet-like structure may be omitted. In the present specification the "first sheet-like structure (or second sheet-like structure)" may be simply referred to as a "sheet-like structure."

<First embodiment>

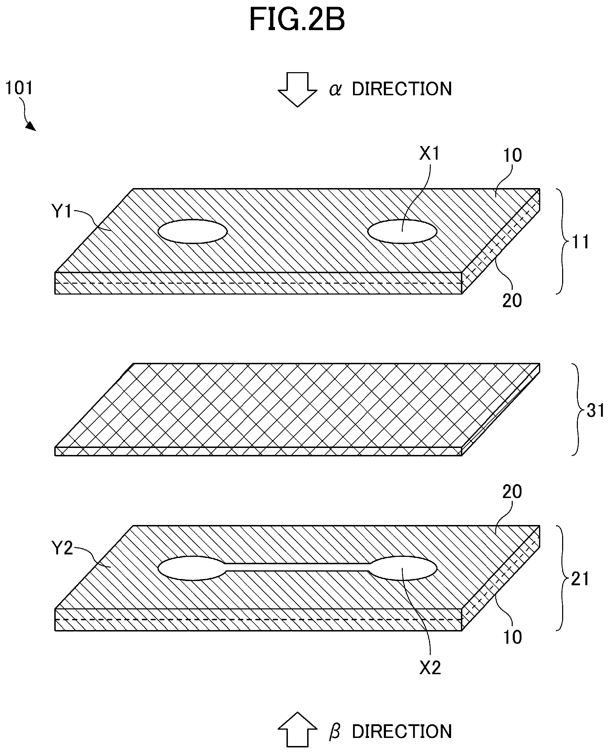

The laminate structure of the present invention may have multiple embodiments other than the embodiment illustrated in Fig. 1. In the present specification, for the simplicity of the description, the details will be described based on the first embodiment illustrated in Figs. 2A and 2B hereinafter.

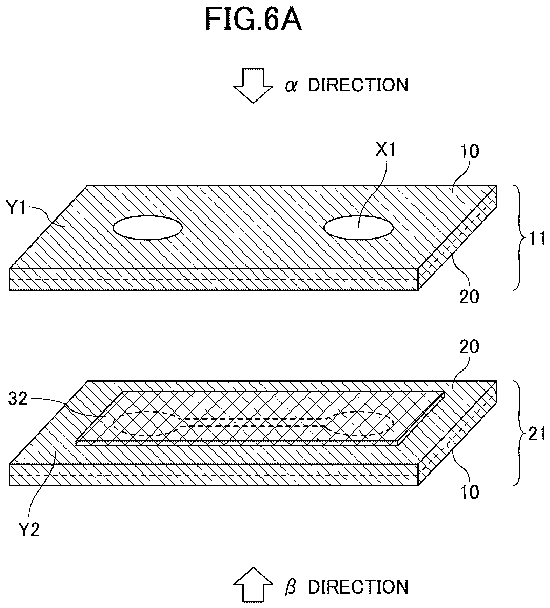

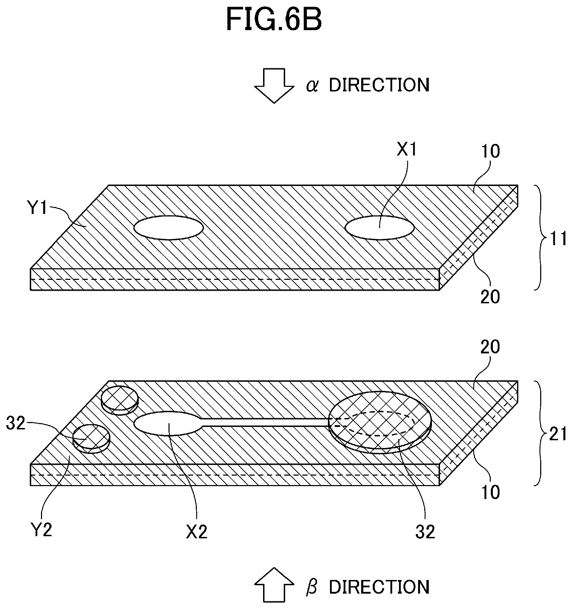

Fig. 2A is a schematic cross-sectional view of the laminate structure of the first embodiment. Fig. 2B is a schematic perspective view of layers constituting the laminate structure of the first embodiment. Note that constituent layers are adjacent to one another and laminated in the laminate structure of the present invention, but constituent layers are illustrated to be separated from one another only in Fig. 2B and below-described Figs. 6A and 6B for the convenience of description.

The laminate structure 101 includes a first sheet-like structure 11 including a flow path X1 and a non-flow path Y1, a second sheet-like structure 21 including a flow path X2 and a non-flow path Y2, and an adhesive layer 31. Although the details will be described later, the adhesive layer 31 is arranged between the first sheet-like structure and the second sheet-like structure to be in contact with part of surfaces of the porous structures to be flow paths such that the flow path (porous structure) of the first sheet-like structure and the flow path (porous structure) of the second sheet-like structure face each other, and the first sheet-like structure and the second sheet-like structure are not separated.

-Sheet-like structure-

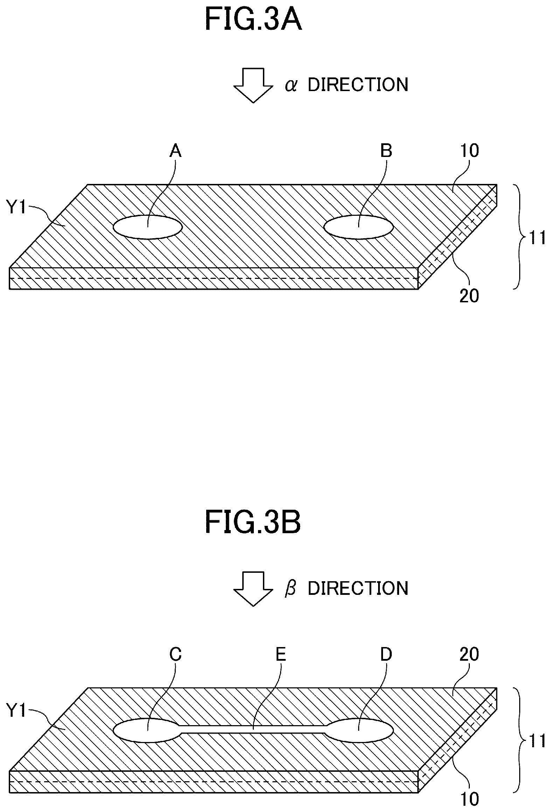

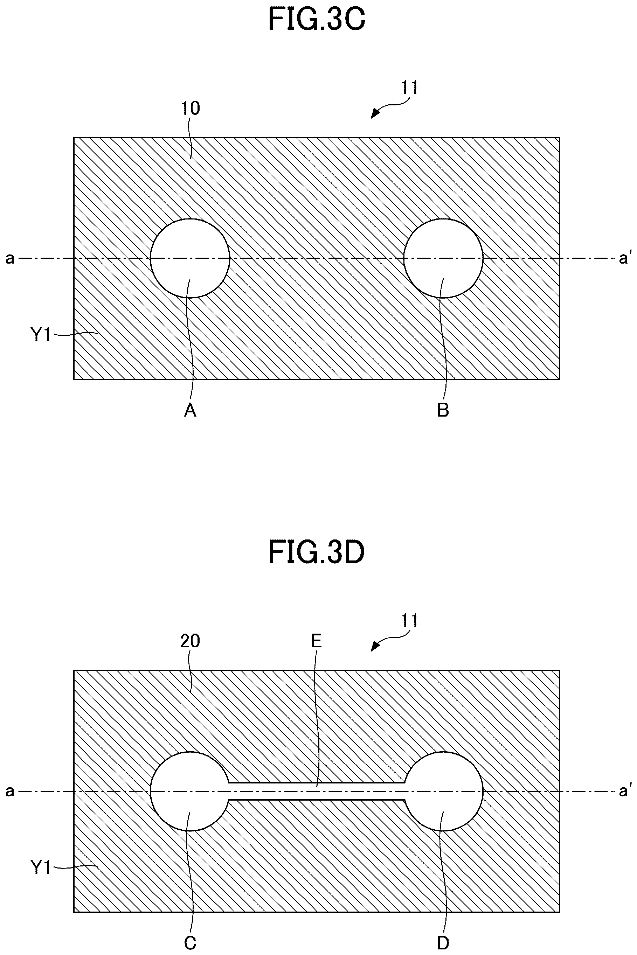

Fig. 3A is a schematic perspective view of the first sheet-like structure of the laminate structure of the first embodiment of Fig. 2A as viewed in the α direction. Fig. 3B is a schematic perspective view of the first sheet-like structure of the laminate structure of the first embodiment of Fig. 2A as viewed in the β direction. Fig. 3C is a schematic plan view of the first sheet-like structure of the laminate structure of the first embodiment of Fig. 2A as viewed in the α direction. Fig. 3D is a schematic plan view of the first sheet-like structure of the laminate structure of the first embodiment of Fig. 2A as viewed in the β direction.

The first sheet-like structure 11 may include a first layer 10 and second layer 20 having mutually different flow path shapes in view of formation of a complex flow path. In the first sheet-like structure, nothing is interposed between the first layer 10 and the second layer 20, and the first layer 10 and the second layer 20 are adjacent to each other. In Fig. 2B, an edge region when a hydrophobic material penetrates from the first layer 10 side is illustrated with a dotted line as an imaginary line. Similarly, an edge region when a hydrophobic material penetrates from the second layer 20 is illustrated with a dotted line as an imaginary line. The imaginary lines are illustrated similarly in other drawings.

In the first layer 10 of the first sheet-like structure, a flow path A, a flow path B, and a non-flow path Y1 that is a region other than the flow paths A and B are provided. The flow path A and the flow path B are separated from each other within the first layer 10 of the first sheet-like structure.

In the second layer 20 of the first sheet-like structure, a flow path C, a flow path D, a flow path E, and a non-flow path Y1 that is a region other than the flow paths C, D, and E are provided. The flow path E is connected to both the flow path C and the flow path D, and a liquid is allowed to flow in the order of the flow path C, the flow path E, and the flow path D, or in the order of the flow path D, the flow path E, and the flow path C.

In the first sheet-like structure 11, the flow path A of the first layer 10 and the flow path C of the second layer 20 are adjacent to each other, and the flow path B of the first layer 10 and the flow path D of the second layer 20 are adjacent to each other. Specifically, in the sheet-like structure 11 of the first embodiment, the flow paths are arranged to be adjacent to one another and connected in the order of the flow path A, the flow path C, the flow path E, the flow path D, and the flow path B, or in the order of the flow path B, the flow path D, the flow path E, the flow path C, and the flow path A.

Each of the flow paths A to E is formed of a porous structure through which a liquid can flow due to a capillary action or the like, and is formed to be exposed at both surfaces of the first sheet-like structure 11 (i.e., the first layer 10 and the second layer 20). The "porous structure" is a structure including multiple communicating pores, and may be generally referred to as a co-continuous structure or a monolithic structure. In the porous structure, continuously connected pores are three-dimensionally spread, thereby allowing a liquid to penetrate (i.e., a capillary action).

A cross-sectional shape of each pore in the porous structure can be appropriately set considering physical properties of a liquid, such as viscosity or the like. Examples of the cross-sectional shape of each pore include substantially circular shapes, substantially elliptical shapes, substantially polygonal shapes, and the like. A size of the pores in the porous structure is not particularly limited, and may be appropriately selected according to the intended purpose. The cross-sectional shapes and size of the pores can be determined, for example, from a cross-sectional photograph captured by a scanning electron microscope (SEM) or the like.

The porosity of the porous structure can be appropriately set considering physical properties of a liquid, such as viscosity, or the like. A method of measuring the porosity is not particularly limited. Examples of the method include a method in which a porous structure is filled with unsaturated fatty acid (commercially available butter), osmium staining is performed, followed by cutting out an inner cross-sectional structure by a focused ion beam (FIB), and a porosity is measured using a SEM, or the like.

The distribution of the pores within the porous structure can be appropriately set considering physical properties of a liquid, such as viscosity or the like, as long as a liquid can flow through the porous structure. The pores are preferably uniformly distributed within the flow path region.

A shape of each of the flow paths A to E in a plan view (i.e., the shape as viewed in the $\alpha$ direction or the $\beta$ direction) is not particularly limited as long as a liquid can flow through the flow path, and may be appropriately selected according to the intended purpose. Examples of the shape include circular shapes, elliptical shapes, square shapes, rectangular shapes, and the like.

A diameter of each of the flow paths A to D is not particularly limited, and may be appropriately selected according to the intended purpose. For example, the diameter can be 3 mm or greater and 10 mm or less. A flow path width of the flow path E is not particularly limited, and may be appropriately selected according to the intended purpose. For example, the flow path width can be 1 mm or greater and 5 mm or less.

The material M of each of the flow paths A to E is not particularly limited as long as the material M has a porous structure through which a liquid can flow, and may be appropriately selected according to the intended purpose. Examples of the material M include paper, such as filter paper, non-woven fabrics, nitrocellulose, polypropylene, and the like. Among the above materials, filter paper is more preferred in view of simplicity and low cost.

The non-flow path Y1 is a region of the first sheet-like structure other than the flow paths A to E, specifically, a region where a liquid flow does not occur. The non-flow path Y1 may be formed of a material M' that is obtained by impregnating the material M with a hydrophobic material.

The hydrophobic material is preferably a material having a melting point of 90°C or lower in view of easy production. The material M' is not particularly limited, and may be appropriately selected according to the intended purpose. Examples of the material M' include wax, compositions including wax, and the like. A viscosity adjusting component (e.g., a resin), dispersion aids, fillers, and the like can be appropriately added to the hydrophobic material.

In the case where the material M is impregnated with the hydrophobic material, the hydrophobic material is preferably melted by heating. The heating temperature can be appropriately set considering a melting point of the hydrophobic material or a melting point of a viscosity adjusting component.

The viscosity of the hydrophobic material when melted can be appropriately set so that the sheet-like structure can be impregnated with the hydrophobic material as desired, considering a thickness, basis weight (density), or the like of the sheet-like structure.

In the case where the material M is impregnated with the hydrophobic material, an impregnation ratio of the hydrophobic material relative to the material M is preferably within the range of 14% or greater and 32% or less.

By producing the sheet-like structure to have the impregnation ratio of 14% or greater, a wall surface (an interface between the material M and the material M') of the flow path for a liquid becomes sufficiently uniform so that a flow of the liquid, for example, from the flow path to another flow path, becomes smooth. By producing the sheet-like structure to have the impregnation ratio of 32% or less, a problem, such as blockage, is sufficiently avoided when the material M is impregnated with the hydrophobic material. Thus, a sheet-like structure having a desired flow path structure can be more assuredly obtained.

The term "impregnation ratio" encompasses an impregnation ratio associated with the material M' in the region of the sheet-like structure, which is formed of the material M' extended over the entire thickness direction. Moreover, the material M' obtained by immersing the material M in a hydrophobic material that is sufficiently heated to have a low viscosity (e.g., heated at 120°C), and leaving the resultant material to stand for a sufficient period (e.g., 3 minutes) while maintaining the temperature is determined as having an impregnation ratio of 100%. The impregnation ratio can be adjusted, for example, by adjusting an amount of the hydrophobic material used for impregnation (a thickness of a hydrophobic film, and the like).

A measurement method of the impregnation ratio is not particularly limited, and may be appropriately selected according to the intended purpose. Examples of the measurement method include the following method.

[Measurement method of impregnation ratio]

Filter paper is cut into an appropriate size, and dried at 120°C for 3 minutes. Then, a dry mass M0 (g) of the filter paper is measured. Subsequently, the filter paper is impregnated with a hydrophobic material, and is left to stand at 120°C for 3 minutes. The impregnated filter paper is held between the same type of filter paper and glass slides, and is left to stand at 120°C for 1 minute with a load of 100 gf to remove the excess hydrophobic material. Thereafter, a mass M1 (g) of the filter paper is measured, and the maximum impregnation amount Pmax (g/m$^2$) per unit area is calculated according to the following equation.

$$\text{Equation: Pmax (g/m}^2\text{)} = (\text{M1} - \text{M0}) \times 1{,}000$$

The viscosity of the hydrophobic material is not particularly limited, and may be appropriately selected according to the intended purpose. For example, for sufficiently avoiding a problem, such as blockage during the impregnation of the material M, the viscosity of the hydrophobic material at 140°C and at the shearing speed of 3,000 s$^{-1}$ is preferably 100 mPa·s or lower, more preferably 50 mPa·s or lower, and yet more preferably 30 mPa·s or lower. Although it is not particularly limited, the viscosity can be measured, for example, by a rheometer (e.g., product name: AR-G2 rheometer, produced by TA Instruments Inc.).

The material M' is preferably colored so that a flow of a liquid can be easily visually recognized, but may be white or transparent, or may not be colored. The coloring of the material M' can be achieved, for example, by impregnating the material M with a colorant in addition to the hydrophobic material. Examples of the colorant include pigments, such as carbon black (a black pigment). The colorant is preferably hydrophobic. Moreover, as the colorant, a colorant that does not adversely affect a reagent used for an inspection or the like is preferably selected.

A shape of the sheet-like structure 11 in a plan view is not particularly limited, and may be appropriately selected according to the intended purpose. For example, the shape of the sheet-like structure 11 may be a rectangular shape as illustrated in Figs. 3A to 3D, or may be a substantially circular shape, a substantially elliptical shape, a substantially rectangular shape, or the like.

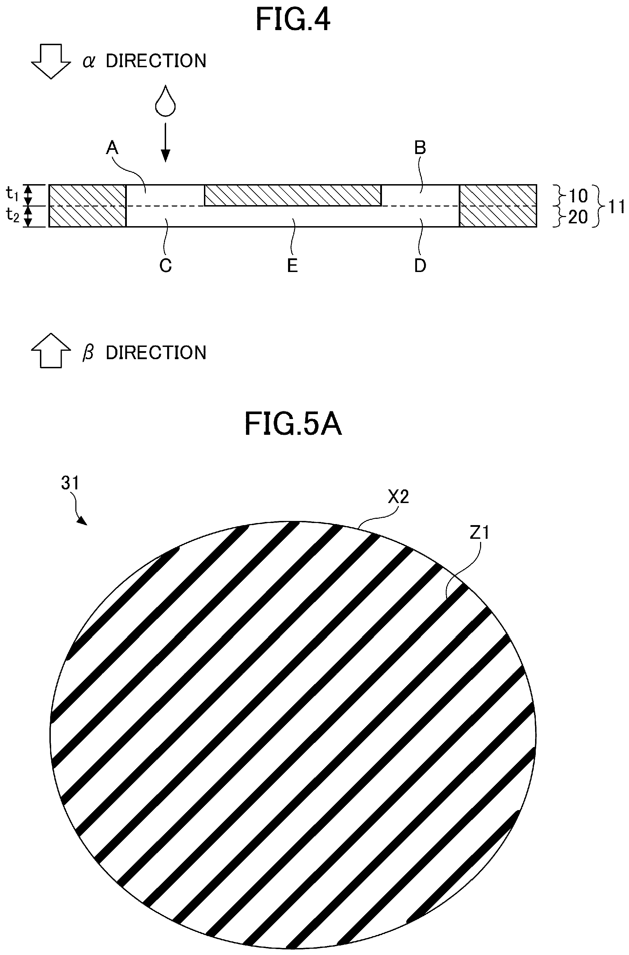

Fig. 4 is a schematic cross-sectional view of the first sheet-like structure of Fig. 3C cut along the line a-a'.

As illustrated in Fig. 4, in the sheet-like structure 11 of the first embodiment, the flow paths are arranged to be adjacent to one another and connected in the order of the flow path A, the flow path C, the flow path E, the flow path D, and the flow path B, or in the order of the flow path B, the flow path D, the flow path E, the flow path C, and the flow path A. In other words, for example, when a liquid is allowed to drip into the flow path A, the sheet-like structure 11 of the first embodiment is configured so that the liquid is passed through the flow path A, the flow path C, the flow path E, and the flow path D in this order, and is ultimately allowed to flow into the flow path B due to a capillary action or the like.

In the first sheet-like structure 11, a ratio (t2/t1) of the average thickness of the second layer 20 (t2) to the average thickness (t1) of the first layer 10 is preferably 0.56 or greater and 2.2 or less. By producing the sheet-like structure to have the average thickness ratio (t2/t1) of 0.56 or greater and 2.2 or less, a problem, such as blockage, can be sufficiently avoided when the material M is impregnated with the hydrophobic material, and a flow speed, speed stability, or both of a liquid, for example, from a flow path to another flow path, can be efficiently improved. From the same viewpoint, the average thickness ratio (t2/t1) is more preferably greater than 1.0, i.e., the average thickness (t2) of the second layer 20 being greater than the average thickness (t1) of the first layer 10, yet more preferably 1.3 or greater, and particularly preferably 1.8 or greater. Moreover, the average thickness ratio (t2/t1) is not particularly limited, and may be set at 3.0 or less.

An average thickness of the first sheet-like structure 11 is not particularly limited, and may be appropriately selected according to the intended purpose. For example, the average thickness of the first sheet-like structure 11 can be 100 μm or greater and 300 μm or less. The average thickness of the first sheet-like structure 11 can be measured, for example, by a thickness gauge under a product name of id-c112bs (produced by Mitutoyo Corporation).

A size of the first sheet-like structure 11 is not particularly limited, and may be appropriately selected according to the intended purpose.

-Adhesive layer-

As illustrated in Fig. 2A, the adhesive layer 31 is arranged between the first sheet-like structure 11 and the second sheet-like structure 21 to be in contact with part of surfaces of the porous structures to be flow paths such that the flow path X1 (porous structure) of the first sheet-like structure and the flow path X2 (porous structure) of the second sheet-like structure face each other, and the first sheet-like structure 11 and the second sheet-like structure 21 are separated.

In the present specification, components constituting the adhesive layer may be collectively referred to as an "adhesive component."

The adhesive layer 31 is preferably water resistant in view of inhibition of leakage of a liquid from flow paths to the outside. The term "water resistant" encompasses characteristics such that a liquid does not penetrate into the adhesive layer, or characteristics such that the adhesive layer is not modified or damaged by a liquid.

An evaluation method of water resistance of the adhesive layer 31 is not particularly limited. For example, the water resistance of the adhesive layer 31 can be evaluated according to JIS K 7114.

The adhesive layer 31 preferably includes a thermal adhesive as an adhesive component. The term "thermal adhesive" encompasses an adhesive having characteristics such that the adhesive is a solid at room temperature, and is melted to become a liquid when heated. An example of the thermal adhesive includes an adhesive that can adhere an adhering target to a target to be adhered by applying a heated and melted thermal adhesive to the adhering target, bringing the target to be adhered and the adhering target into contact with each other with the thermal adhesive being interposed between the adhering target and the target to be adhered, followed by cooling (or returning to room temperature).

The thermal adhesive included in the adhesive layer 31 as an adhesive component preferably includes a hot-melt resin having a melting point of 80°C or higher and 120°C or lower.

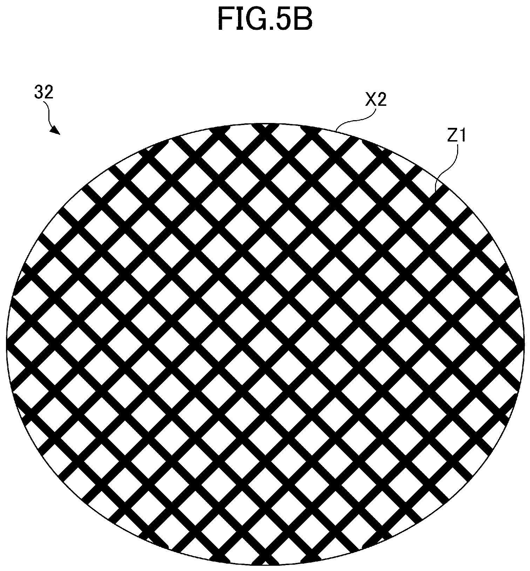

Fig. 5A is a schematic plan view illustrating an example of the adhesive layer of the laminate structure of the first embodiment as viewed in the $\alpha$ direction. Fig. 5B is a schematic plan view illustrating another example of the adhesive layer of the laminate structure of the first embodiment as viewed in the $\alpha$ direction. For avoiding complicated illustration, only the flow path X2 (porous structure) of the second sheet-like structure is illustrated in Figs. 5A and 5B, and others are omitted.

The adhesive layer 31 is not arranged on an entire surface of the flow path X2 (porous structure) in the sheet-like structure, but is arranged to be in contact with part of the surface of the flow path X2 (porous structure).

A shape of the adhesive layer 31 is preferably a shape where an adhesive component Z1 is arranged in the shape of parallel lines on the flow path X2 (porous structure) as illustrated in Fig. 5A. The "shape of parallel lines" includes a shape of substantially parallel lines that are apparently parallel. Moreover, the shape of the adhesive layer is more preferably a shape (mesh form) where the adhesive component Z1 is arranged so that portions of the adhesive component Z1 cross one another on the flow path X2 (porous structure), as illustrated in Fig. 5B.

In the present specification, the adhesive layer in the shape where the adhesive component is arranged in the form of parallel lines on the flow path is referred to as the "adhesive layer in the form of parallel lines," and the shape where the adhesive component is arranged to be crossed on the flow path is referred to as the "adhesive layer in the mesh form."

In the adhesive layer, the adhesive component may be arranged at regular intervals or at irregular intervals.

In the case where the adhesive layer is the mesh form, within a region in the flow paths connected via the adhesive layer, a region where the porous structure of the first sheet-like structure and the porous structure of the second sheet-like structure communicate with each other is referred to as an "opening." Specifically, the term "opening" encompasses a portion through which a liquid can flow from the porous structure of the first sheet-like structure to the porous structure of the second sheet-like structure (or from the porous structure of the second sheet-like structure to the porous structure of the first sheet-like structure) due to a capillary action or the like. An area of each "opening" is referred to as an "opening area."

In the case where the adhesive layer is a mesh form, within the flow paths connected via the adhesive layer, a region of the adhesive layer that is in contact with both the porous structure of the first sheet-like structure and the porous structure of the second sheet-like structure is referred to as a "linking portion," and an area of the "linking portion" is referred to as a "linking area."

In the present specification, multiple "openings" and the "linking portion" are collectively described as a "connection portion" and an area of the "connection portion" is described as a "connection area."

The shape of each opening in the adhesive layer is not particularly limited, and may be appropriately set according to physical properties of a liquid, such as viscosity. Examples of the shape of the opening include quadrangles (squares, rectangles, rhombuses, and the like), circles (perfect circles, ellipses, and the like), polygons, and the like.

The average value of the opening area (mm$^2$) per opening of the adhesive layer is not particularly limited, and may be appropriately set according to physical properties of a liquid, such as viscosity. The average value of the opening area (mm$^2$) per opening is preferably 0.2 mm$^2$. In particular, the average value of the opening area (mm$^2$) per opening of the adhesive layer is preferably 1/5 or less relative to the average value of the connection area (mm$^2$) in view of excellent flowability.

Examples of a method of measuring the opening area (mm$^2$) per opening of the adhesive layer include a method in which a laminate structure is immersed in a liquid, such as water, to swell layers constituting the laminate structure to separate the layers, and the opening area is measured using a microscope (produced by KEYENCE CORPORATION). The average value of the opening area (mm$^2$) per opening of the adhesive layer can be determined, for example, by measuring opening areas of adjacent 10 openings and calculating an average value of the measured values.

The average thickness of the adhesive layer is not particularly limited, and may be appropriately selected according to the intended purpose. The average thickness of the adhesive layer is preferably 50 $\mu$m or greater and 200 $\mu$m or less. A measuring method of the average thickness is not particularly limited. For example, the average thickness can be measured by a thickness gauge under a product name of id-c112bs (produced by Mitutoyo Corporation) or the like.

Fig. 6A is a schematic perspective view for explaining an example of a distribution of the adhesive layer in the laminate structure of the first embodiment. Fig. 6B is a schematic perspective view for explaining another example of the distribution of the adhesive layer in the laminate structure of the first embodiment.

As described above, the adhesive layer may be arranged in any manner as long as the adhesive layer is arranged between the first sheet-like structure and the second sheet-like structure to be in contact with at least part of surfaces of the porous structures so that the porous structure of the first sheet-like structure and the porous structure of the second sheet-like structure face each other, and the first sheet-like structure and the second sheet-like structure are not separated. For example, the adhesive layer may be arranged over an entire surface of one side of the sheet-like structure as illustrated in Fig. 2B, may be arranged over the entire flow path exposed at the surface of the sheet-like structure on one side as illustrated in Fig. 6A, or may be arranged over only part of the flow path exposed at the surface of the sheet-like structure on one side as illustrated in Fig. 6B. Note that the arrangement of the adhesive layer is preferably appropriately adjusted so that the first sheet-like structure and the second sheet-like structure are not partially separated from each other, as illustrated in Fig. 6B.

Instead of the adhesive layer, a thermal adhesive sheet, in which an adhesive layer is arranged on a substrate, may be used. The thermal adhesive sheet preferably includes the adhesive layer in which the thermal adhesive (adhesive component) is arranged in the shape of parallel lines or in a mesh form on the substrate having multiple opening. Further, the adhesive layer is more preferably an adhesive layer in which the thermal adhesive (adhesive component Z1) is arranged in a mesh form on the substrate Z2 having multiple openings.

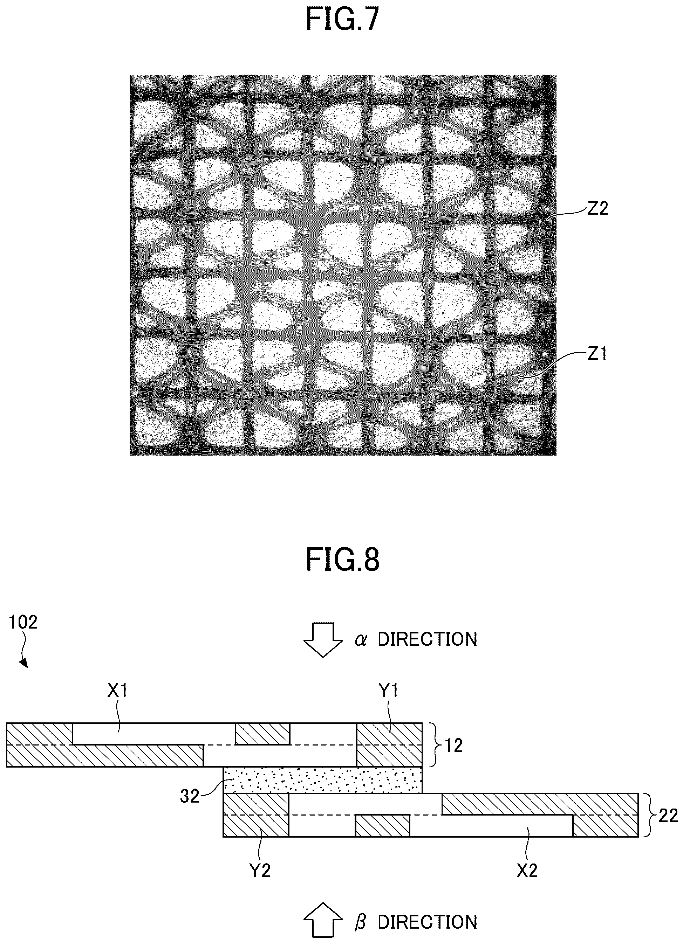

As an example of the thermal adhesive sheet, the thermal adhesive sheet illustrated in Fig. 7 is exemplified. Fig. 7 is an image illustrating an example of the thermal adhesive sheet of the present invention. In the image illustrated in Fig. 7, the adhesive component Z1 is arranged diagonally with respect to each opening of the substrate Z2 above the substrate Z2 having openings in the mesh form having grids in the longitudinal direction and the width direction.

Note that the openings of the substrate of the thermal adhesive sheet are different from the openings of the above-described adhesive layer.

Use of the thermal adhesive sheet including the substrate as the adhesive layer is preferred because physical strength of the laminate structure itself can be improved.

A material of the substrate of the thermal adhesive sheet is not particularly limited, and may be appropriately selected according to the intended purpose. Examples of the material include polyester and the like. In the case where the thermal adhesive sheet is a mesh form, examples of the material of the substrate include polyester, nylon, cotton, and the like.

A size and the number of openings in the substrate of the thermal adhesive sheet are not particularly limited, as long as the thermal adhesive sheet has a configuration capable of bonding sheet-like structures together, and may be appropriately set according to the intended purpose. For example, the openings of the substrate can be 30-mesh (850 $\mu$m) to 120-mesh (200 $\mu$m).

A shape of each opening of the substrate of the thermal adhesive sheet is not particularly limited, and may be appropriately set according to the intended purpose. Examples of the shape of the opening include substantially circular shapes, substantially elliptical shapes, substantially rectangular shapes, and the like.

The average thickness of the thermal adhesive sheet is not particularly limited, and may be appropriately selected according to the intended purpose. For avoiding problems, such as adhesion failures or impaired flowability, the average thickness of the thermal adhesive sheet is preferably 50 $\mu$m or greater and 150 $\mu$m or less.

The thermal adhesive sheet may be, for example, a microporous adhesive sheet obtained by performing fine punching (punching process) on an adhesive sheet that does not have openings in both a substrate and an adhesive layer. The size and number of the openings in the microporous adhesive sheet may be the same as the substrate of the above-described thermal adhesive sheet.

The punching (punching process) can be performed, for example, using a rotary die cutter. For example, after forming an adhesive layer on release paper, punching is performed. The adhesive layer is bonded onto one side of a constituent material, followed by peeling off the release paper, and the other side of the adhesive layer can be bonded.

<Second embodiment>

Fig. 8 is a schematic cross-sectional view illustrating layers constituting a laminate structure of a second embodiment.

The laminate structure 102 illustrated in Fig. 8 is the same as the laminate structure 101 of the first embodiment, except that the flow path structure of the sheet-like structure is different from the flow path structure of the sheet-like structure of the first embodiment.

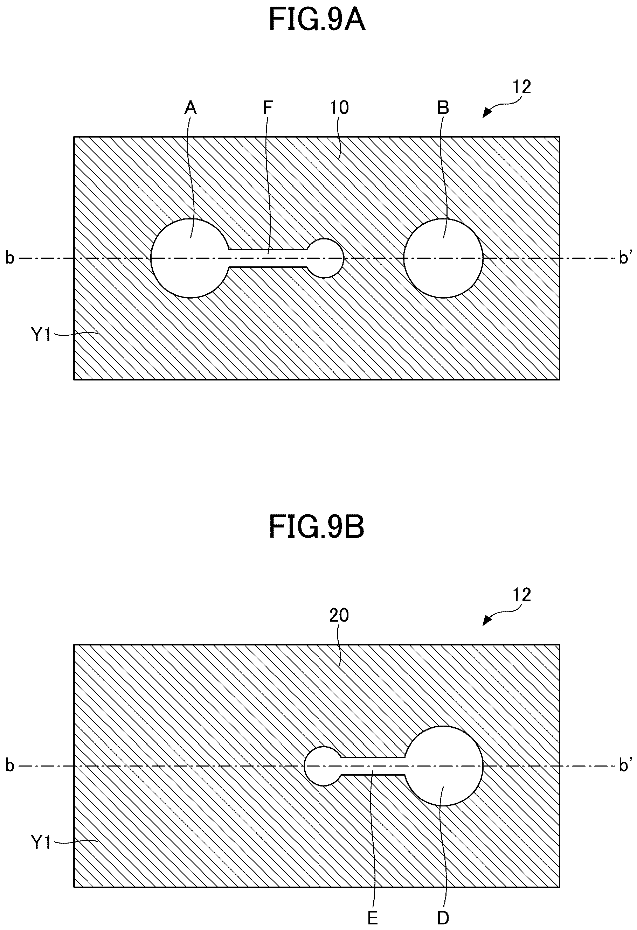

Fig. 9A is a schematic plan view of the first sheet-like structure of the laminate structure of the second embodiment of Fig. 8 as viewed in the $\alpha$ direction. Fig. 9B is a schematic plan view of the first sheet-like structure of the laminate structure of the second embodiment of Fig. 8 as viewed in the $\beta$ direction.

As illustrated in Fig. 9A, in the first sheet-like structure 12 of the second embodiment, a flow path A, a flow path B, a flow path F connected to the flow path A, and a non-flow path Y1 that is a region other than the flow path F are provided in the first layer 10. The flow path A and the flow path B are separated from each other in the first layer 10 of the first sheet-like structure.

As illustrated in Fig. 9B, in the first sheet-like structure 12 of the second embodiment, a flow path D, a flow path E, and a non-flow path Y1 that is a region other than the flow paths E and D are provided in the second layer 20, where the flow path E is connected to the flow path D.

In the first sheet-like structure 12 of the second embodiment, the flow path B of the first layer 10 and the flow path D of the second layer 20 are adjacent to each other, and the flow path F of the first layer 10 is connected to the flow path E of the second layer 20. Specifically, in the first sheet-like structure 12 of the second embodiment, the flow paths are arranged to be adjacent to one another and are connected in the order of the flow path A, the flow path F, the flow path E, the flow path D, and the flow path B, or in the order of the flow path B, the flow path D, the flow path E, the flow path F, and the flow path A.

In addition, the flow path A, the flow path B, the flow path D, the flow path E, the flow path F, and the non-flow path Y1 are formed of the material M or material M' described in the above section of <First embodiment>.

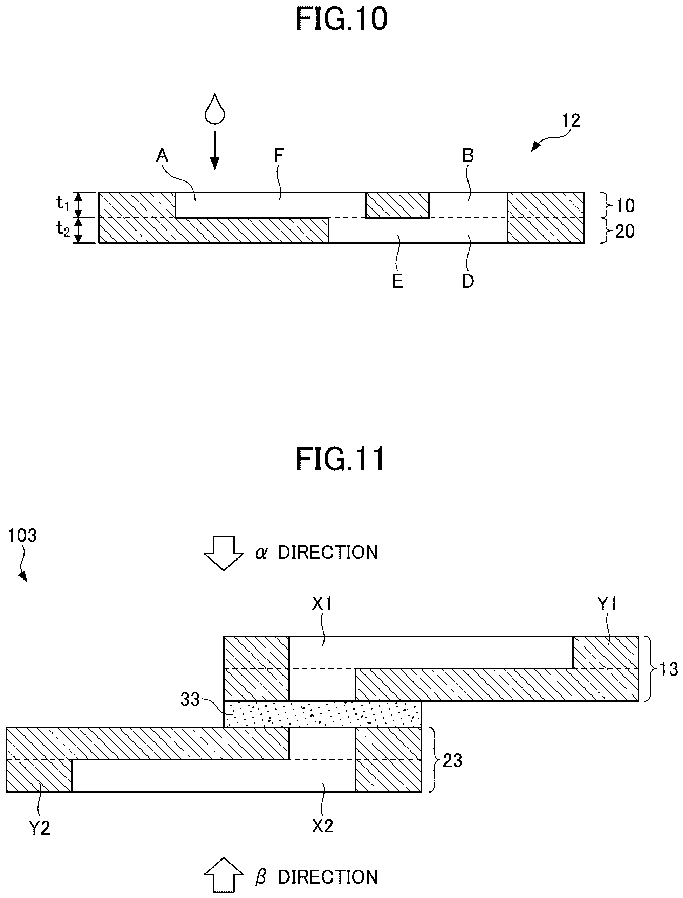

Fig. 10 is a schematic cross-sectional view of the first sheet-like structure of Fig. 9A cut along the line b-b'.

As illustrated in Fig. 10, the first sheet-like structure 12 of the second embodiment is configured, for example, such that when a liquid is allowed to drip into the flow path A, the liquid is passed through the flow path A, the flow path F, the flow path E, and the flow path D in this order due to a capillary action or the like, and is ultimately allowed to flow into the flow path B.

Other than above, descriptions of the same features as in the first embodiment are omitted.

<Third embodiment>

Fig. 11 is a schematic cross-sectional view of a laminate structure of a third embodiment.

The laminate structure 103 illustrated in Fig. 11 is the same as the laminate structure 101 of the first embodiment, except that the flow path structure of the sheet-like structure is different from the flow path structure of the sheet-like structure of the first embodiment.

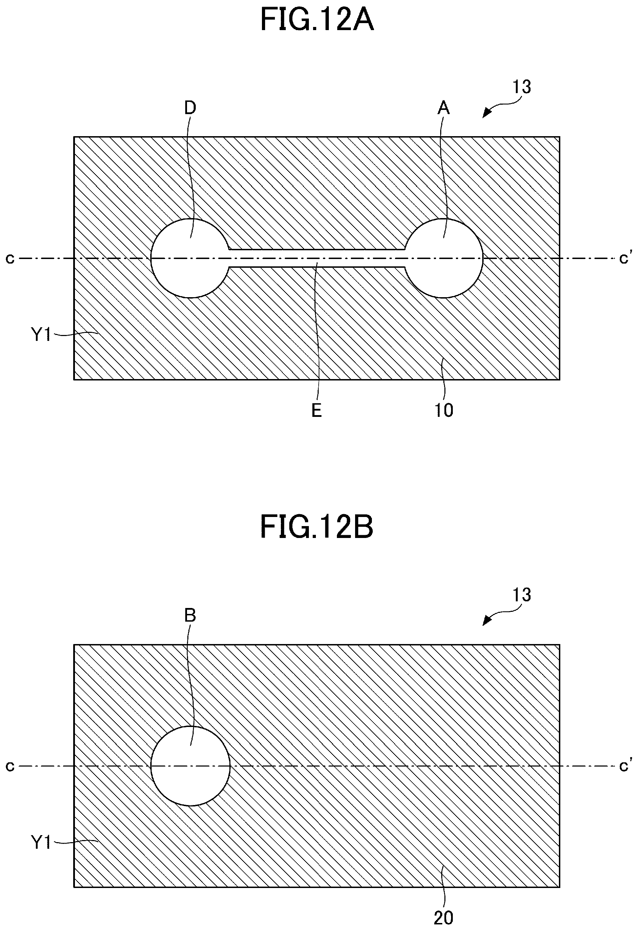

Fig. 12A is a schematic plan view of the first sheet-like structure of the laminate structure of the third embodiment of Fig. 11 as viewed in the $\alpha$ direction. Fig. 12B is a schematic plan view of the first sheet-like structure of the laminate structure of the third embodiment of Fig. 11 as viewed in the $\beta$ direction.

In the first sheet-like structure 13 of the third embodiment, a flow path A, a flow path D, a flow path E, and a non-flow path Y1 that is a region other than the flow paths A, E, and D are provided in the first layer 10 as illustrated in Fig. 12A. The flow path E is connected to both the flow path D and the flow path A.

In the first sheet-like structure 13 of the third embodiment, a flow path B and a non-flow path Y1 that is a region other than the flow path B are provided in the second layer 20, and a flow path A is not provided, as illustrated in Fig. 12B.

In the first sheet-like structure 13 of the third embodiment, the flow path D of the first layer 10 and the flow path B of the second layer 20 are adjacent to each other. Specifically, in the first sheet-like structure 13 of the third embodiment, the flow paths are arranged to be adjacent to one another and are connected in the order of the flow path A, the flow path E, the flow path D, and the flow path B, or in the order of the flow path B, the flow path D, the flow path E, and the flow path A.

Moreover, the flow path A, the flow path B, the flow path D, the flow path E, and the non-flow path Y1 are formed of the material M or material M' described in the above section of

<First embodiment>.

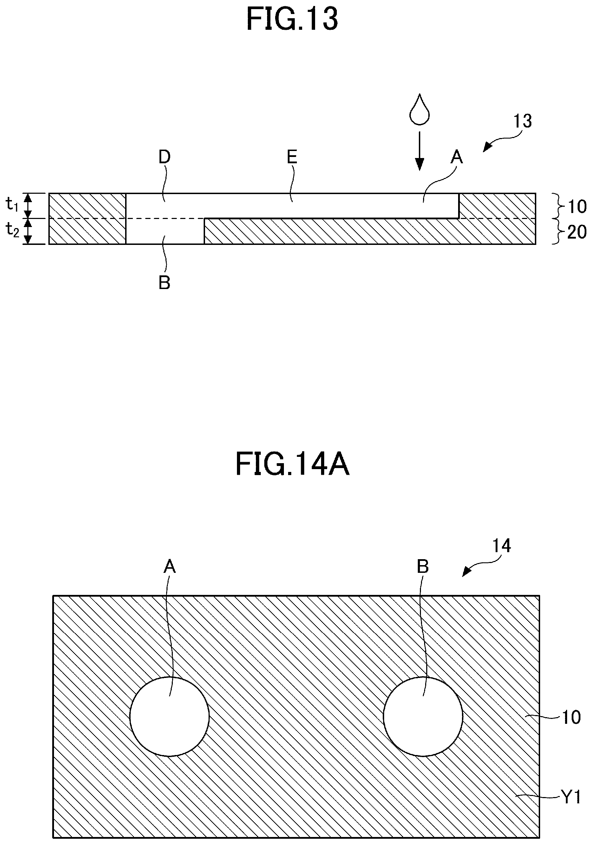

Fig. 13 is a schematic cross-sectional view of the first sheet-like structure of Fig. 12A cut along the line c-c'.

As illustrated in Fig. 13, the first sheet-like structure 13 of the third embodiment is configured, for example, such that when a liquid is allowed to drip into the flow path A, the liquid is passed through the flow path A, the flow path E, and the flow path D in this order due to a capillary action or the like, and is ultimately allowed to flow into the flow path B.

Other than above, descriptions of the same features as in the first embodiment are omitted.

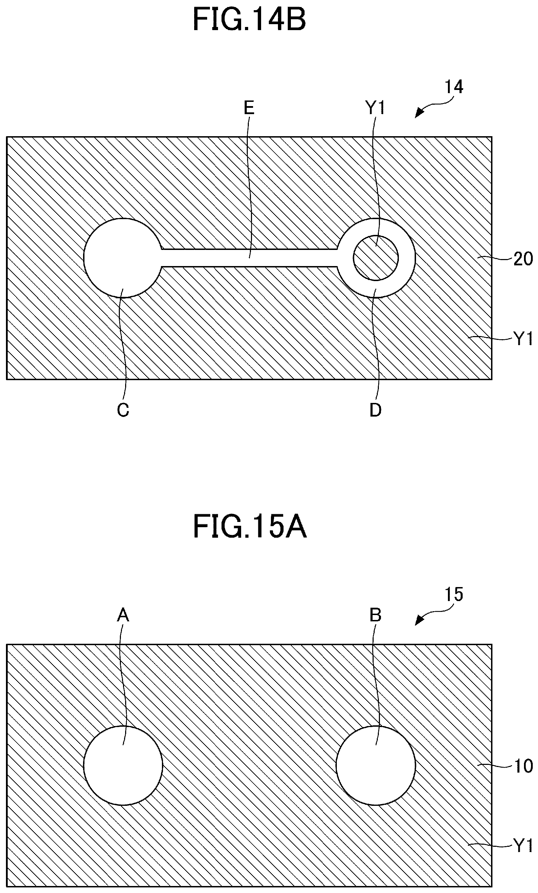

Fig. 14A is a schematic plan view of the first sheet-like structure of the fourth embodiment of the laminate structure of the fourth embodiment as viewed in the $\alpha$ direction. Fig. 14B is a schematic plan view of the first sheet-like structure of the fourth embodiment of the laminate structure of the fourth embodiment as viewed in the $\beta$ direction.

As illustrated in Figs. 14A and 14B, the first sheet-like structure 14 of the third embodiment is the same as illustrated in Figs. 3C and 3D, except that the flow path D formed in the second layer 20 is an annular structure, and a non-flow path Y1 is formed inside the annular structure.

The flow path D, which is an annular structure, may have an arbitrary outline shape, such as a circular shape, an elliptical shape, a rectangular shape, or the like. The flow path D preferably has an outline shape substantially matched with the flow path B in a plan view of the sheet-like structure. Moreover, the non-flow path Y1 formed inside the flow path D preferably has a shape obtained by reducing the outline shape of the flow path D in a plan view of the sheet-like structure.

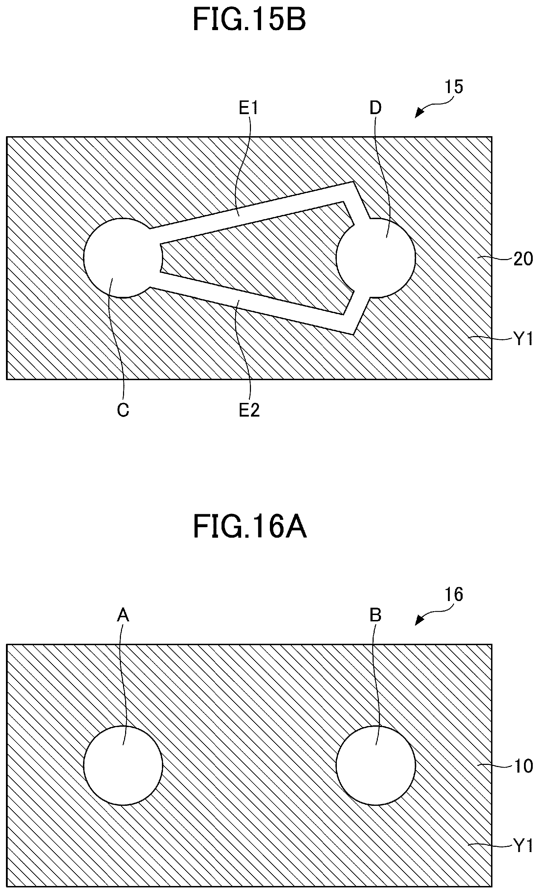

Fig. 15A is a schematic plan view of the first sheet-like structure of the fifth embodiment of the laminate structure of the fifth embodiment as viewed in the α direction. Fig. 15B is a schematic plan view of the first sheet-like structure of the fifth embodiment of the laminate structure of the fifth embodiment as viewed in the β direction.

As illustrated in Figs. 15A and 15B, the first sheet-like structure 15 of the fifth embodiment is the same as in Figs. 3C and 3D, except the first sheet-like structure 15 of the fifth embodiment has the structure in which the second layer 20 has multiple flow paths E (two paths E1 and E2 in Fig. 15B). With regard to the above structure, multiple flow paths F are provided to match the number of the flow paths E in the case of the first sheet-like structure 12 of the second embodiment, so that the multiple flow paths F of the first layer 10 and the multiple flow paths E of the second layer 20 can be connected, respectively.

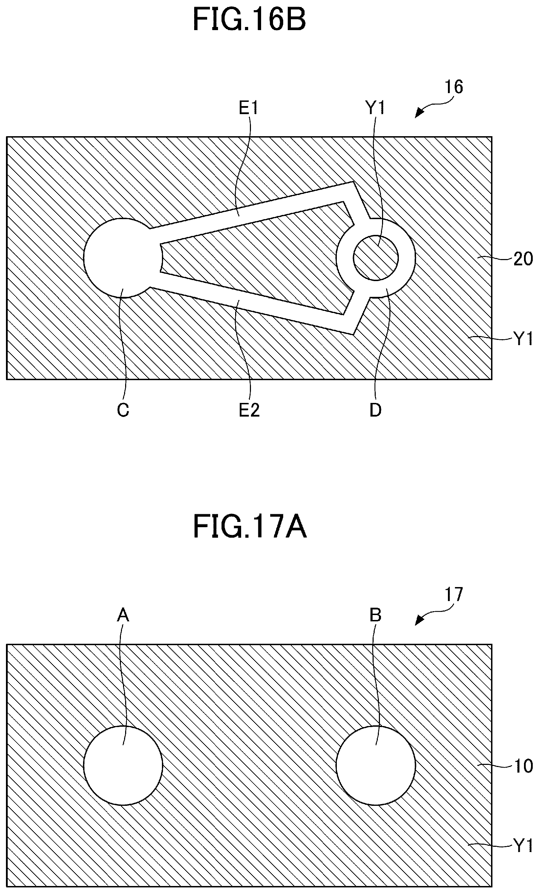

Fig. 16A is a schematic plan view of the first sheet-like structure of the sixth embodiment of the laminate structure of the sixth embodiment as viewed in the α direction. Fig. 16B is a schematic plan view of the first sheet-like structure of the sixth embodiment of the laminate structure of the sixth embodiment as viewed in the β direction.

As illustrated in Figs. 16A and 16B, the first sheet-like structure 16 of the sixth embodiment has a structure combining the structures of Figs. 14A, 14B, 15A, and 15B.

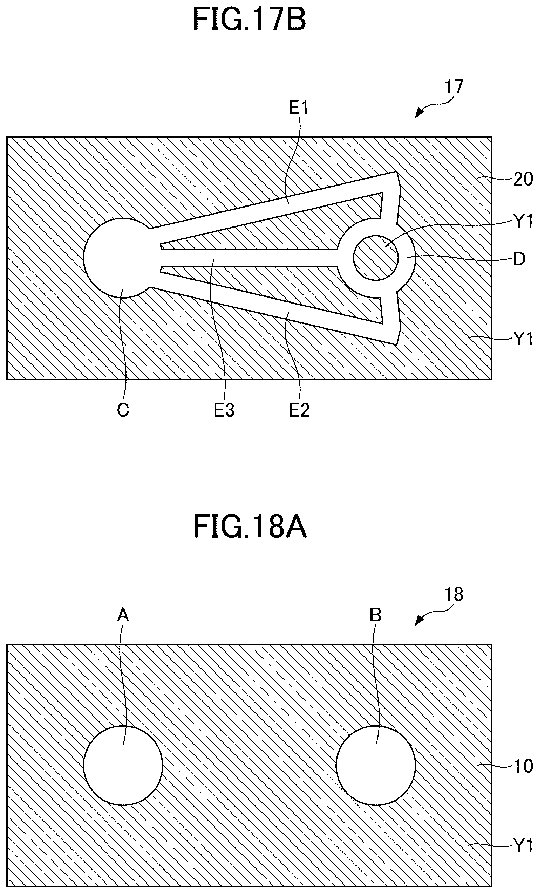

Fig. 17A is a schematic plan view of the first sheet-like structure of the seventh embodiment of the laminate structure of the seventh embodiment as viewed in the α direction. Fig. 17B is a schematic plan view of the first sheet-like structure of the seventh embodiment of the laminate structure of the seventh embodiment as viewed in the β direction.

As illustrated in Figs. 17A and 17B, the first sheet-like structure 17 of the seventh embodiment is substantially the same as in Fig. 16B except that the first sheet-like structure 17 of the seventh embodiment has a structure in which the second layer 20 has three flow paths E (E3, in addition to E1 and E2). In the sheet-like structure 17 illustrated in Fig. 17B, the three flow paths E are connected to the flow path D in a manner such that the three flow paths E face one another.

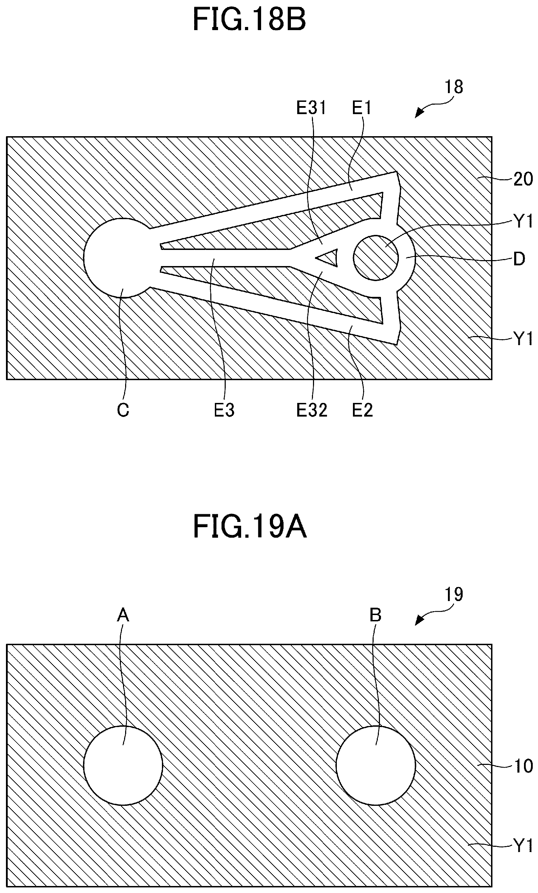

Fig. 18A is a schematic plan view of the first sheet-like structure of the eighth embodiment of the laminate structure of the eighth embodiment as viewed in the α direction. Fig. 18B is a schematic plan view of the first sheet-like structure of the eighth embodiment of the laminate structure of the eight embodiment as viewed in the β direction.

As illustrated in Figs. 18A and 18B, the first sheet-like structure 18 of the eighth embodiment is substantially the same as in Fig. 17B, except that the first sheet-like structure 18 of the eighth embodiment has a structure in which the flow path E3 is branched into two (E31 and E32) that are connected to the flow path D.

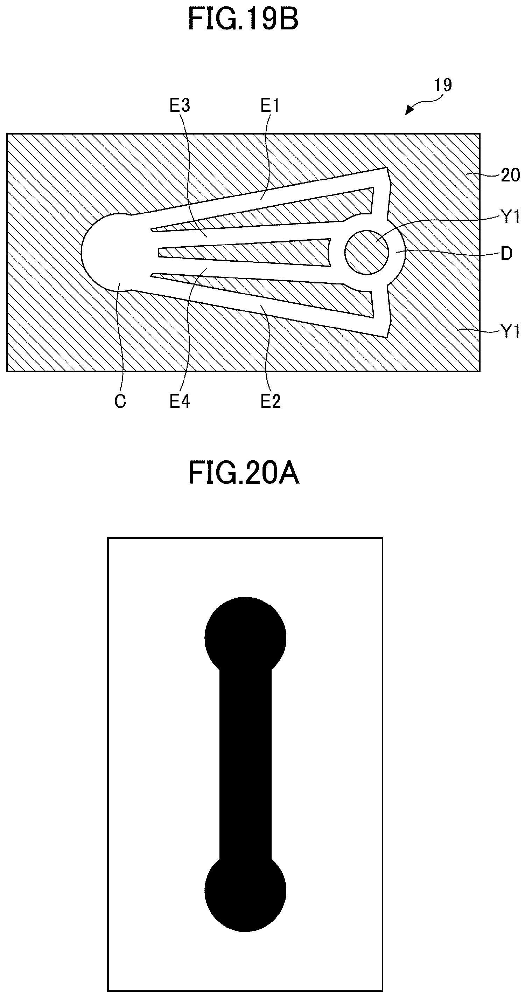

Fig. 19A is a schematic plan view of the first sheet-like structure of the ninth embodiment of the laminate structure of the ninth embodiment as viewed in the α direction. Fig. 19B is a schematic plan view of the first sheet-like structure of the ninth embodiment of the laminate structure of the ninth embodiment as viewed in the β direction.

As illustrated in Figs. 19A and 19B, the first sheet-like structure 19 of the ninth embodiment is substantially the same as in Fig. 17B, except that the first sheet-like structure 19 of the ninth embodiment has a structure having two flow paths E (E3 and E4) in addition to the flow path E1 and the flow path E2. In the sheet-like structure 19 illustrated in Fig. 19B, the four flow paths E are connected to the flow path D in a manner such that the four flow paths E face one another.

The number of the flow paths E (and the flow paths F) in the sheet-like structure as illustrated in Figs. 14A to 19B is preferably 4 or less, more preferably 3 or less, and yet more preferably 2 in view of inhibition of increase in a liquid amount. Moreover, the number of connection points of the flow paths E to the flow path D is preferably 4 or less, more preferably 3 or less, and yet more preferably 2.

Among the multiple flow paths E, at least two flow paths E are preferably connected to the flow path D in a manner such that the flow paths E connected to the flow path D face each other. Moreover, among the multiple flow paths E, at least two flow paths E preferably have substantially the same shape.

The above-described sheet-like structure can be produced, for example, by forming a predetermined portion (flow path B and the like) in a sheet-like material to produce a first layer 10, forming a predetermined portion (flow path D and the like) in another sheet-like material to produce a second layer 20, and laminating the above two sheet-like materials. Alternatively, the above-described sheet-like structure 1 can be also produced by forming a predetermined portion in a part of one sheet-like material to produce a first layer 10, forming a predetermined portion in another part of the sheet-like material to produce a second layer 20, and folding the sheet-like material while adjusting the positions of the first layer and the second layer.

The sheet-like structure of the present invention is preferably produced by forming a first layer on one side of a sheet-like material, and forming a second layer on the other side of the sheet-like material. The above sheet-like structure in which the first layer and the second layer are respectively formed on corresponding sides of the sheet-like material has various advantages, such as (1) the labor and cost for laminating (or folding) can be avoided, (2) a flow of a liquid between the first layer and the second layer by a capillary action can be ensured, (3) disposal is easily performed because a tool for retaining the laminate (folding) of the sheet-like materials or the like is not necessary, and the like.

As a specific production method of the sheet-like structure, for example, the sheet-like structure can be produced by the following method.

[One example of method of producing sheet-like structure]

First, a hydrophobic material, a colorant, and a resin are blended. The resultant mixture is melted and mixed, for example, at 100°C or higher and 140°C or lower, thereby preparing a wax ink. The wax ink is applied onto a substrate, such as a polyethylene terephthalate film, thereby producing an ink ribbon. Next, a predetermined flow path shape is printed on high quality paper by a thermal transfer printer (product name: L'esprit R412v-ex, produced by SATO HOLDINGS CORPORATION), thereby forming missing flow path patterns in the printed portions of the ink ribbon. After fixing pieces of the ink ribbon having the missing flow path patterns on corresponding front and back sides of filter paper, the resultant stack is passed through a thermal laminator (e.g., product name: GL535ML, produced by GBG) set at a predetermined temperature and line speed to transfer the wax ink to the filter paper and allow the wax ink to penetrate into the filter paper to form a three-dimensional flow path, thereby producing a sheet-like structure.

The "predetermined temperature and line speed" are not particularly limited as long as the predetermined temperature and line speed are conditions under which the wax ink can penetrate and can be transferred to the filter paper. For transferring, the conditions can be set at 85°C and at the line speed of 10 mm/sec. For permeation, the conditions can be set at 85°C and at the line speed of 5 mm/sec.

(Inspection device)

The laminate structure of the present invention can be suitably used as an inspection device. Examples of the inspection device include pregnancy test kits, inspection devices using a measuring method called an immunochromatography method in which the principle of sandwich ELISA and the principle of chromatography are combined, and the like.

(Method of producing laminate structure and apparatus of producing laminate structure)

<First embodiment>

The method of producing the laminate structure of the present invention according to a first embodiment includes an adhesive layer formation step, a transfer step, and a bonding step, and may further include other steps, as necessary.

The apparatus of producing the laminate structure in association with the method of producing the laminate structure of the present invention according to the first embodiment includes an adhesive layer formation unit, a transfer unit, and a bonding unit, and may further include other units, as necessary.

The method of producing the laminate structure is suitably performed by the apparatus of producing the laminate structure.

-Adhesive layer formation step and adhesive layer formation unit-

The adhesive layer formation step is a step of arranging a thermal adhesive in a shape of parallel lines on a release substrate to form an adhesive layer. In other words, the adhesive layer formation step is a step of forming an adhesive layer in a shape of parallel lines on a release substrate.

The adhesive layer formation unit is a unit that arranges a thermal adhesive in a shape of parallel lines on a release substrate to form an adhesive layer. In other words, the adhesive layer formation unit is a unit that forms an adhesive layer in a shape of parallel lines on a release substrate.

The adhesive layer formation step is suitably performed by the adhesive layer formation unit.

In the adhesive layer formation step and by the adhesive layer formation unit, an adhesive layer in which a thermal adhesive is arranged in a mesh form on the release substrate is preferably formed. In other words, a mesh form adhesive layer is preferably formed on the release substrate in the adhesive layer formation step and by the adhesive layer formation unit.

Since the thermal adhesive and the adhesive layer are the same as the thermal adhesive and the adhesive layer described in the above section of (Laminate structure), redundant description will be omitted.

A material of the release substrate is not particularly limited as long as an adhesive layer formed on the release substrate can be released in a later step, and may be appropriately selected according to the intended purpose. Examples of the material include release paper, styrene films, and the like.

A size, shape, and structure of the release substrate are not particularly limited as long as an adhesive layer formed on the release substrate can be released in a later step, and may be appropriately selected according to the intended purpose.

Examples of a method of applying the thermal adhesive to the release substrate include a coating method using a T-die, and the like.

-Transfer step and transfer unit-

The transfer step of the method of producing the laminate structure of the present invention according to the first embodiment is a step of transferring the adhesive layer to a first sheet-like structure having a porous structure serving as a flow path exposed at a surface of the first sheet-like structure.

The transfer unit used in the method of producing the laminate structure of the present invention according to the first embodiment is a unit that transfers the adhesive layer to a first sheet-like structure having a porous structure serving as a flow path exposed at a surface of the first sheet-like structure.

The transfer step is suitably performed by the transfer unit.

Note that the first sheet-like structure in the transfer step may be the second sheet-like structure.

A method of transferring the adhesive layer to the first sheet-like structure is not particularly limited, and may be appropriately selected according to the intended purpose. For example, the adhesive layer can be transferred to the first sheet-like structure by a laminator (GL535ML, produced by GBC) under the conditions of 120°C and 5 mm/sec.

-Bonding step and bonding unit-

The bonding step of the method of producing the laminate structure of the present invention according to the first embodiment is a step of removing the release substrate, and bonding a second sheet-like structure having a porous structure serving as a flow path exposed at a surface of the second sheet-like structure.

The bonding step of the method of producing the laminate structure of the present invention according to the first embodiment is a unit that removes the release substrate and bonds a second sheet-like structure having a porous structure serving as a flow path exposed at a surface of the second sheet-like structure.

The bonding step is suitably performed by the bonding unit.

Note that the second sheet-like structure in the transfer step may be the first sheet-like structure.

At the time of bonding the second sheet-like structure, bonding is performed in a manner such that the porous structure of the first sheet-like structure and the porous structure of the second sheet-like structure face each other. The temperature and line speed of the bonding can be appropriately adjusted according to a melting point of a thermal adhesive to be used. For example, laminating can be performed under the conditions of 80°C or higher and 150°C or lower, and 5 mm/sec. For laminating, for example, a laminator (GL535ML, produced by GBC) or the like can be used.

-Other steps and other units-

Other steps of the method of producing the laminate structure of the present invention according to the first embodiment are not particularly limited, and may be appropriately selected according to the intended purpose. Examples of the other steps include a pretreatment step and the like.

Other units used in the method of producing the laminate structure of the present invention according to the first embodiment are not particularly limited, and may be appropriately selected according to the intended purpose. Examples of the other units include a pretreatment unit and the like.

The above other steps are suitably performed by the above other units.

The pretreatment step is a step of coating the adhesive layer with a surfactant. The pretreatment unit is a unit that coats the adhesive layer with a surfactant.

The pretreatment step and the pretreatment unit are preferred because a problem such that a liquid having a high surface tension cannot pass through the adhesive layer can be solved.

The surfactant is not particularly limited, and may be appropriately selected according to the intended purpose. Examples of the surfactant include Tweeen 20 (Polyoxyethylene sorbitan monolaurate) and the like.

The pretreatment method, specifically, the method of coating the adhesive layer with the surfactant is not particularly limited, and may be appropriately selected according to the intended purpose. Examples of the pretreatment method include a method in which a laminate structure or a sheet-like structure is immersed in an aqueous solution of the surfactant, followed by drying, and the like.

The pretreatment step may be performed after bonding the sheet-like structures to produce a laminate structure, or may be performed before bonding the sheet-like structures. In the case where the pretreatment step is performed after producing the laminate structure, the pretreatment step is preferably performed before producing an inspection device (e.g., before addition of a reagent or the like).

<Second embodiment>

The method of producing the laminate structure of the present invention according to a second embodiment includes a transfer step and a bonding step, and may further include other steps, as necessary.

The apparatus of producing the laminate structure in association with the method of producing the laminate structure of the present invention according to the second embodiment includes a transfer unit and a bonding unit, and may further include other units, as necessary.

The method of producing the laminate structure is suitably performed by the apparatus of producing the laminate structure.

-Transfer step and transfer unit-

The transfer step of the method of producing the laminate structure of the present invention according to the second embodiment is a step of transferring a thermal adhesive sheet, which includes a substrate having multiple openings, and an adhesive layer in which a thermal adhesive is arranged in a shape of parallel lines on the substrate, to a first sheet-like structure having a porous structure serving as a flow path exposed at a surface of the first sheet-like structure.

The transfer unit used in the method of producing the laminate structure of the present invention according to the second embodiment is a unit that transfers a thermal adhesive layer, which includes a substrate having multiple openings and an adhesive layer in which a thermal adhesive is arranged in a shape of parallel lines on the substrate, to a first sheet-like structure having a porous structure serving as a flow path exposed at a surface of the first sheet-like structure.

The transfer step is suitably performed by the transfer unit.

Note that the first sheet-like structure in the transfer step may be the second sheet-like structure.

The first sheet-like structure and the thermal adhesive sheet in the transfer step are the same as the first sheet-like structure and the thermal adhesive sheet described in the above section of (Laminate structure), and therefore redundant description will be omitted.

A method of transferring the thermal adhesive sheet to the first sheet-like structure is the same as the transferring method described in the above section of <First embodiment>, and therefore redundant description will be omitted.

-Bonding step and bonding unit-

The bonding step of the method of producing the laminate structure of the present invention according to the second embodiment is a step of bonding a second sheet-like structure having a porous structure serving as a flow path exposed at a surface of the second sheet-like structure.

The bonding unit used in the method of producing the laminate of the present invention according to the second embodiment is a unit that bonds a second sheet-like structure having a porous structure serving as a flow path exposed at a surface of the second sheet-like structure.

The bonding step is suitably performed by the bonding unit.

The second sheet-like structure in the bonding step is the same as the second sheet-like structure described in the above section of (Laminate structure), and therefore redundant description will be omitted.

A method of bonding to the second sheet-like structure is the same as the bonding method described in the above section of

<First embodiment> except that the substrate is not removed, and therefore redundant description will be omitted.

The transfer step of the second embodiment and the bonding step of the second embodiment may be performed simultaneously. In this case, the first sheet-like structure, the thermal adhesive sheet, and the second sheet-like structure are stacked in this order, and laminated, for example, under conditions of 120°C and 5 mm/sec. For laminating, for example, a laminator (GL535ML, produced by GBC) or the like can be used.

-Other steps and other units-

The other steps of the method of producing the laminate structure of the present invention according to the second embodiment are not particularly limited, and may be appropriately selected according to the intended purpose. Examples of the other steps include a pretreatment step.

The other units used in the method of producing the laminate structure of the present invention according to the second embodiment are not particularly limited, and may be appropriately selected according to the intended purpose. Examples of the other units include a pretreatment unit.

The above other steps are suitably performed by the above other units.

Note that the other steps and the other units are the same as the other steps and the other units described in the above section of <First embodiment>, and therefore redundant description will be omitted.

(Thermal adhesive sheet for sheet-like structure and method of using thermal adhesive sheet for sheet-like structure)

The thermal adhesive sheet of the present invention can be suitably used as a thermal adhesive sheet for a sheet-like structure including a porous structure serving as a flow path exposed at a surface of the sheet-like structure. In other words, the thermal adhesive sheet for the sheet-like structure of the present invention is a thermal adhesive sheet for a sheet-like structure, which is used for a sheet-like structure having a porous structure serving as a flow path exposed at a surface of the sheet-like structure, and the thermal adhesive sheet for the sheet-like structure includes a substrate having multiple openings, and an adhesive layer in which a thermal adhesive is arranged in a mesh form on the substrate.

The method of using the thermal adhesive sheet for the sheet-like structure of the present invention is a usage method of the thermal adhesive sheet for the sheet-like structure, in which the thermal adhesive sheet is used for a sheet-like structure having a porous structure serving as a flow path exposed at a surface of the sheet-like structure, and the thermal adhesive sheet for the sheet-like structure includes a substrate having multiple openings, and an adhesive layer in which a thermal adhesive is arranged in a mesh form on the substrate.

The thermal adhesive sheet for the sheet-like structure can be transferred to and bonded to a sheet-like structure having a porous structure serving as a flow path exposed at a surface of the sheet-like structure (first sheet-like structure or second sheet-like structure).

The thermal adhesive sheet for the sheet-like structure is the same as the thermal adhesive sheet described in the above section of (Laminate structure), and therefore redundant description will be omitted.

Examples

Next, the present invention will be more specifically described through Examples and Comparative Examples, but the present invention is not limited to Examples below.

<Production of laminate structure>

-Production of ink ribbon-

The following materials were blended, and the resultant mixture was melted and mixed at 100°C to prepare a wax ink. Paraffin wax as a hydrophobic material (product name: Paraffin Wax-135, produced by NIPPON SEIRO CO., LTD.): 72.0 parts by mass Synthetic wax as a hydrophobic material (product name: DIACARNA™ 30, produced by Mitsubishi Chemical Corporation): 18.0 parts by mass

Carbon black as a colorant (product name: MA-100, produced by Mitsubishi Chemical Corporation): 1.8 parts by mass
Resin (product name: Ultrathene® 722, produced by Tosoh Corporation): 11.25 parts by mass

The viscosity of the obtained wax ink under the conditions of 140°C and the shearing speed of 3,000 s$^{-1}$ was 23 mPa·s. Note that the viscosity was measured by Rheometer AR-G2 (produced by TA Instruments Inc.).

The obtained wax ink was applied onto a polyethylene terephthalate film having the average thickness of 6 $\mu$m (product name: Lumirror® #6C F531, produced by TORAY INDUSTRIES, INC.) so that the average thickness of the applied ink was to be 10 $\mu$m, thereby producing an ink ribbon.

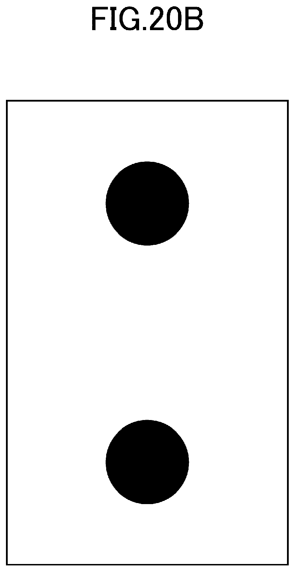

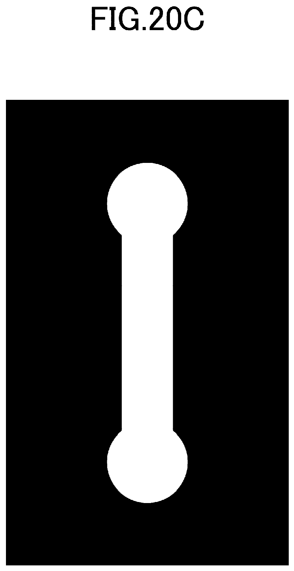

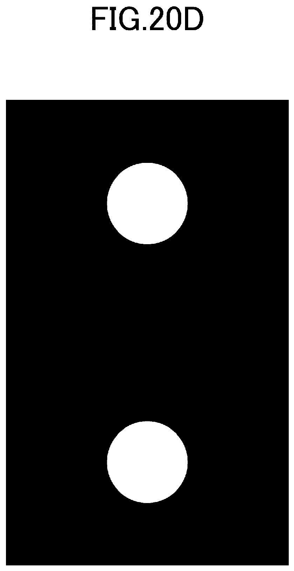

The flow path shapes illustrated in Figs. 20A and 20B were printed on high quality paper by a thermal transfer printer (product name: L'esprit R412v-ex, produced by SATO HOLDINGS CORPORATION), thereby forming missing flow path patterns in the printed portions of the ink ribbon (see Figs. 20C and 20D).

-Confirmation of flow path formation-

First, whether a flow path was appropriately formed was checked.

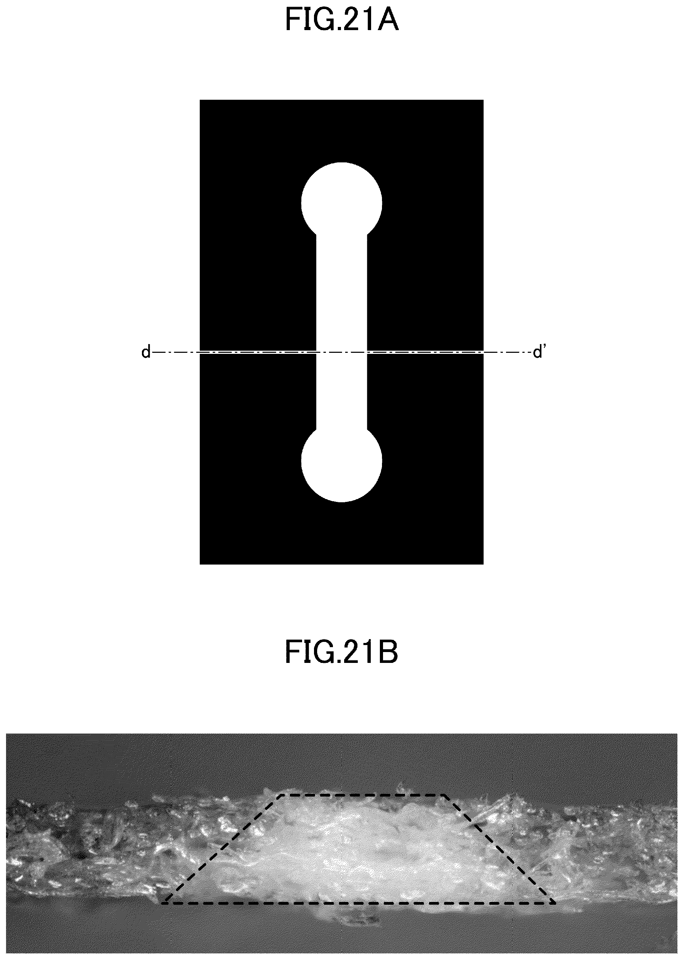

The flow path pattern illustrated in Fig. 21A was printed on one side of filter paper (product name: Whatman #41) with the wax ink using a printer (product name: Xerox ColorQube8570, Xerox Corporation). Thereafter, the printed filter paper was heated in an oven under the conditions of 120°C for 2 minutes to allow the wax ink to penetrate into the filter paper, thereby forming a single-side printed flow path. After impregnating the single-side printed flow path with a fluorescent ink (fluorescent marker pen, produced by ASKUL Corporation) aqueous solution, the filter paper was cut at the position corresponding to the line d-d' of Fig. 21A, and a cross-sectional photograph of the flow path was captured by a microscope (produced by KEYENCE CORPORATION). The result is presented in Fig. 21B.

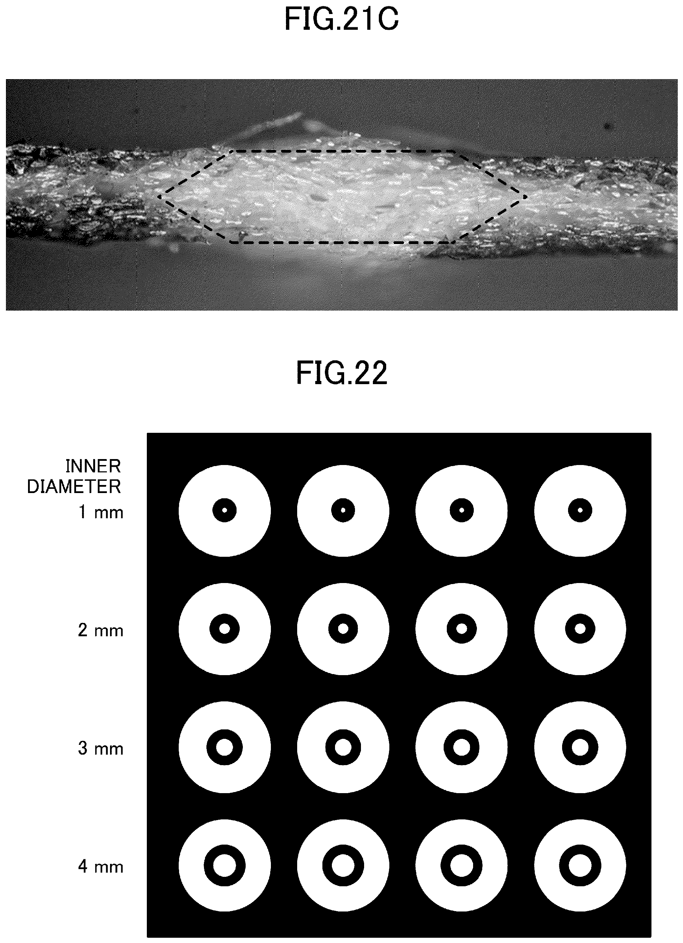

Similarly, a double-side printed flow path was formed within filter paper by performing impregnation under the same conditions, except that the flow path pattern illustrated in Fig. 21A was printed on both sides of filter paper (product name: Whatman #41). After impregnating the double-side printed flow path with a fluorescent ink (fluorescent marker pen, produced by ASKUL Corporation) aqueous solution, the filter paper was cut at the position corresponding to the line d-d' of Fig. 21A, and a cross-sectional photograph of the flow path was captured by a microscope (produced by KEYENCE CORPORATION). The result is presented in Fig. 21C.

The fluorescent ink was only confirmed in the portions each surrounded with the dotted line in Figs. 21B and 21C, and leakage of the fluorescent ink could not be observed in other regions. Therefore, it was confirmed that the flow path was appropriately formed. Since the single-side printing was performed, namely the wax ink penetrated from one side of the filter paper in Fig. 21B, the formed flow path had the shape that expanded from the plane from which the wax ink penetrated toward the plane of the opposite side, namely, a tapered shape. Since double-side printing was performed, namely, the wax ink penetrated from both sides of the filter paper in Fig. 21C, the formed flow path became symmetric, and had a diamond shape.

-Production of sheet-like structure-

The pieces of the ink ribbon in which the missing flow path patterns illustrated in Figs. 20C and 20D were respectively formed were fixed on corresponding front and back sides of filter paper, respectively. Thereafter, the resultant stack was passed through a thermal laminator (product name: GL535ML, produced by GBG) set at a predetermined temperature so that the wax ink was transferred to and penetrated into the filter paper to form a three-dimensional flow path within the filter paper, thereby producing a sheet-like structure. The "predetermined temperature" is 85°C with the line speed of 10 mm/sec for transferring, and 85°C with the line speed of 5 mm/sec for permeation.

The obtained sheet-like structure had the configuration illustrated in Fig. 4.

-Production of laminate structure-

A thermal adhesive sheet (product name: SP7600HF, produced by Dexerials Corporation) and 2 sheets of the obtained sheet-like structures were stacked in a manner such that the thermal adhesive sheet was interposed between the two sheet-like structures. The resultant stacked layers were laminated by a thermal laminator (product name: GL535ML, produced by GBG) at 120°C and at 5 mm/sec, thereby producing a laminate structure. At the time of laminating, the laminating was performed so that the porous structure of one sheet-like structure and the porous structure of the other sheet-like structure faced each other as illustrated in Fig. 1.

The used thermal adhesive sheet (product name: SP7600HF, produced by Dexerials Corporation) included a mesh form adhesive layer on a mesh form substrate (material: polyester).

The obtained laminate structure had the configuration illustrated in Fig. 1.

<Evaluation on flowability of flow path>

A fluorescent ink (fluorescent marker pen, produced by ASKUL Corporation) aqueous solution (12 μL) was added from one end of the flow path of the obtained laminate structure, and whether the solution reached the opposite end of the flow path was confirmed. It was confirmed that the aqueous fluorescent ink was spread across the entire area of the flow path within the laminate structure. Moreover, leakage of the fluorescent ink aqueous solution from the flow path to the outside was checked by a UV lamp (product name: BL-LED3435-UV, produced by The Contec Group), but the leakage of the fluorescent ink aqueous solution was not confirmed.

<Evaluation on liquid passage within adhesive layer>

A sheet-like structure having a flow path having an inner diameter of 1 mm to 4 mm and a width of 3 mm was produced in the same manner as the above "-Production of sheet-like structure-" except that the configuration of the flow path of the sheet-like structure was changed to a doughnut shape as illustrated in Fig. 22. The obtained sheet-like structure, a thermal adhesive sheet (product name: SP7600HF, mesh form, produced by Dexerials Corporation), and a transparent polyester film (product name: Lumirror S10-188μ, produced by TORAY INDUSTRIES, INC.) were stacked in this order, and laminated at 120°C and at 5 mm/sec, thereby producing each of evaluation samples 1 to 4.

A solution (5 μL) in which an aqueous fluorescent ink was dissolved in distilled water was added to a center portion (inner diameter portion) of each of the obtained evaluation samples 1 to 4, and the sample was left to stand for 5 minutes. After leaving the sample to stand, the sample was visually observed from the transparent polyester film side, and the presence and absence of the liquid passed to the polyester film interface, and the presence and absence of leakage of the liquid from the flow path to the outside were confirmed. The results are presented in Table 1.

[Table 1]

| | Evaluation sample | | | |
|---|---|---|---|---|
| | 1 | 2 | 3 | 4 |
| Inner diameter of flow path (mm) | 1 | 2 | 3 | 4 |
| Presence or absence of liquid leakage | Absent | Absent | Absent | Absent |
| Flowability (n=4) | 2/4 | 4/4 | 4/4 | 4/4 |
| Opening area ratio | 4 | 15.7 | 35 | 62.8 |

A ratio (opening area ratio) of the connection area to the opening area per opening in Table 1 above was calculated according to the following equation.

opening area ratio = connection area $(mm^2)$/opening area $(mm^2)$                                    Equation:

Note that each opening in the thermal adhesive sheet (product name: SP7600HF, mesh form, produced by Dexerials Corporation) was approximately 450 $\mu$m $\times$ 450 $\mu$m, and the opening area was calculated as 0.2 $mm^2$ from the average value of measured opening areas of ten adjacent openings.

It was confirmed that the solution reached the PET film interface via the thermal adhesive sheet, and no liquid leakage from the flow path to the outside was confirmed in all of the evaluation samples. Moreover, it could be confirmed that the liquid passage was also excellent when the opening area ratio was 5 or greater.

The present application claims priority based on Japanese Patent Application No. 2022-188117, filed on November 25, 2022, and Japanese Patent Application No. 2023-188110, filed on November 2, 2023, the entire content of which are incorporated herein by reference.

REFERENCE SIGNS LIST

100     laminate structure
101     laminate structure of first embodiment
102     laminate structure of second embodiment
103     laminate structure of third embodiment
1       first sheet-like structure
10      first layer
20      second layer
11      first sheet-like structure of first embodiment
12      first sheet-like structure of second embodiment
13      first sheet-like structure of third embodiment
14      first sheet-like structure of fourth embodiment
15      first sheet-like structure of fifth embodiment
16      first sheet-like structure of sixth embodiment
17      first sheet-like structure of seventh embodiment
18      first sheet-like structure of eighth embodiment
19      first sheet-like structure of ninth embodiment
2       second sheet-like structure
21      second sheet-like structure of first embodiment
3       adhesive layer
31      adhesive layer
32      adhesive layer
33      adhesive layer
A       flow path
B       flow path
C       flow path
D       flow path
E       flow path

E1      flow path
E2      flow path
E3      flow path
E31     flow path
E32     flow path
E4      flow path
X1      flow path
X2      flow path
Y1      non-flow path
Y2      non-flow path
M       material
M'      material

## Claims

1.  A laminate structure comprising:

    a first sheet-like structure having a porous structure serving as a flow path, the porous structure being exposed at a surface of the first sheet-like structure;
    a second sheet-like structure having a porous structure serving as a flow path, the porous structure being exposed at a surface of the second sheet-like structure; and
    an adhesive layer that is arranged between the first sheet-like structure and the second sheet-like structure and is in contact with part of a surface of the porous structure of the first sheet-like structure and part of a surface of the porous structure of the second sheet-like structure in a manner such that the porous structure of the first sheet-like structure and the porous structure of the second sheet-like structure face each other, and the first sheet-like structure and the second sheet-like structure are not separated.

2.  The laminate structure according to claim 1,
    wherein the adhesive layer is water resistant.

3.  The laminate structure according to claim 1 or 2,
    wherein the adhesive layer is in a shape of parallel lines.

4.  The laminate structure according to any one of claims 1 to 3,
    wherein the adhesive layer is in a mesh form.

5.  The laminate structure according to any one of claims 1 to 4,
    wherein a ratio of a connection area of the flow path to an opening area per opening of the adhesive layer is 5 or greater.

6.  The laminate structure according to any one of claims 1 to 5,
    wherein the adhesive layer includes a thermal adhesive.

7.  The laminate structure according to claim 6,
    wherein the laminate structure includes a thermal adhesive sheet that includes a substrate having multiple openings, and the adhesive layer in which the thermal adhesive is arranged in a mesh form on the substrate.

8.  The laminate structure according to claim 7,
    wherein the substrate is in a mesh form.

9.  An inspection device comprising:
    the laminate structure of any one of claims 1 to 8.

10. A method of producing the laminate structure of any one of claims 1 to 8, the method comprising:

    an adhesive layer formation step of arranging a thermal adhesive in a shape of parallel lines to form the adhesive layer on a release substrate;
    a transfer step of transferring the adhesive layer onto the first sheet-like structure having the porous structure serving as a flow path exposed at the surface of the first sheet-like structure; and

a bonding step of removing the release substrate, and bonding the second sheet-like structure having the porous structure serving as a flow path exposed at the surface of the second sheet-like structure.

**11.** The method of producing the laminate structure according to claim 10, wherein the adhesive layer formation step includes arranging the thermal adhesive in a mesh form to form the adhesive layer.

**12.** A method of producing the laminate structure of any one of claims 1 to 8, the method comprising:

a transfer step of transferring a thermal adhesive sheet onto the first sheet-like structure having the porous structure serving as a flow path exposed at the surface of the first sheet-like structure, the thermal adhesive sheet including a substrate having multiple openings, and the adhesive layer in which a thermal adhesive is arranged in a shape of parallel lines on the substrate; and
a bonding step of bonding the second sheet-like structure having the porous structure serving as a flow path exposed at the surface of the second sheet-like structure.

**13.** The method of producing the laminate structure according to claim 12, wherein the thermal adhesive sheet includes the substrate, and the adhesive layer in which the thermal adhesive is arranged in a mesh form on the substrate.

**14.** A thermal adhesive sheet for a sheet-like structure comprising:

a substrate having multiple openings; and
an adhesive layer in which a thermal adhesive is arranged in a mesh form on the substrate,

wherein the thermal adhesive sheet for the sheet-like structure is used for a sheet-like structure having a porous structure serving as a flow path exposed at a surface of the sheet-like structure.

**15.** A method of using a thermal adhesive sheet for a sheet-like structure, the method comprising:
using the thermal adhesive sheet for the sheet-like structure for a sheet-like structure having a porous structure serving as a flow path exposed at a surface of the sheet-like structure,
wherein the thermal adhesive sheet for the sheet-like structure includes a substrate having multiple openings, and an adhesive layer in which a thermal adhesive is arranged in a mesh form on the substrate.

# FIG.1

100

⬇ α DIRECTION

Y1   X1

3

X2   Y2

1

2

⬆ β DIRECTION

# FIG.2A

101

⬇ α DIRECTION

X1   Y1

31

Y2   X2

11

21

⬆ β DIRECTION

# FIG.2B

101

⬇ α DIRECTION

X1    10

Y1

11

20

31

20

Y2

21

10

X2

⬆ β DIRECTION

# FIG.3A

⬇ α DIRECTION

A                B     10

Y1

11

20

# FIG.3B

⬇ β DIRECTION

C    E    D    20

Y1

11

10

# FIG.3C

# FIG.3D

## FIG.4

## FIG.5A

# FIG.5B

# FIG.6A

α DIRECTION

X1 10

Y1

11

20

20

32

21

10

Y2

β DIRECTION

# FIG.6B

⬇ α DIRECTION

X1　　10

Y1

11

20

20

32

21

10

Y2　　　　X2　　　　　　　　32

⬆ β DIRECTION

# FIG.7

# FIG.8

# FIG.9A

A    F    10    B    12

b ——————— b'

Y1

# FIG.9B

20    12

b ——————— b'

Y1

E    D

# FIG.10

# FIG.11

# FIG.12A

# FIG.12B

# FIG.13

# FIG.14A

# FIG.14B

# FIG.15A

# FIG.15B

# FIG.16A

# FIG.16B

# FIG.17A

# FIG.17B

# FIG.18A

# FIG.18B

# FIG.19A

# FIG.19B

# FIG.20A

# FIG.20B

# FIG.20C

# FIG.20D

# FIG.21A

d—·—·—·—·—·—·—·—·—·—·—·—·—·—·—d'

# FIG.21B

# FIG.21C

# FIG.22

## INTERNATIONAL SEARCH REPORT

International application No.

**PCT/JP2023/039970**

| A. | CLASSIFICATION OF SUBJECT MATTER |
|---|---|

*G01N 35/08*(2006.01)i; *B81B 1/00*(2006.01)i; *B81C 1/00*(2006.01)i; *G01N 37/00*(2006.01)i
FI:   G01N35/08 A; G01N37/00 101; B81B1/00; B81C1/00

According to International Patent Classification (IPC) or to both national classification and IPC

| B. | FIELDS SEARCHED |
|---|---|

Minimum documentation searched (classification system followed by classification symbols)

G01N35/08; G01N37/00; B81B1/00; B81C1/00; F28F3/12

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT | |
|---|---|---|

| Category* | Citation of document, with indication, where appropriate, of the relevant passages | Relevant to claim No. |
|---|---|---|
| X | JP 2003-190768 A (KAWAMURA INST OF CHEM RES) 08 July 2003 (2003-07-08) paragraphs [0070], [0072], [0092], [0098], [0109], [0116], [0119], [0126], [0142]-[0144], [0158]-[0160], fig. 4 | 1-15 |
| A | JP 2005-098790 A (CSTEC KK) 14 April 2005 (2005-04-14) | 1-15 |
| A | JP 2006-030160 A (HITACHI MAXELL LTD) 02 February 2006 (2006-02-02) | 1-15 |
| A | US 2020/0071160 A1 (EPIGEM LIMITED) 05 March 2020 (2020-03-05) | 1-15 |
| A | JP 2017-001119 A (ASAHI FR R&D CO LTD) 05 January 2017 (2017-01-05) | 1-15 |

☐ Further documents are listed in the continuation of Box C.      ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **28 December 2023** | **16 January 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

**PCT/JP2023/039970**

| Patent document cited in search report | | | Publication date (day/month/year) | Patent family member(s) | | | Publication date (day/month/year) |
|---|---|---|---|---|---|---|---|
| JP | 2003-190768 | A | 08 July 2003 | (Family: none) | | | |
| JP | 2005-098790 | A | 14 April 2005 | (Family: none) | | | |
| JP | 2006-030160 | A | 02 February 2006 | US | 2005/0272142 | A1 | |
| US | 2020/0071160 | A1 | 05 March 2020 | GB | 2557587 | A | |
| | | | | WO | 2018/104752 | A1 | |
| JP | 2017-001119 | A | 05 January 2017 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021175970 A
- JP 2022018036 A
- JP 2022188117 A
- JP 2023188110 A

**Non-patent literature cited in the description**

- **WHITESIDE et al.** *Analytical Chemistry*, 01 January 2010, vol. 82 (1)
- **QUOC TRUNG HUA et al.** *analytical sciences*, April 2019, vol. 35